(19) Europäisches Patentamt
European Patent Office
Office européen des brevets

(11) Veröffentlichungsnummer: **0 034 122**
**B1**

# (12) EUROPÄISCHE PATENTSCHRIFT

(45) Veröffentlichungstag der Patentschrift: **21.09.83**

(51) Int. Cl.³: **C 07 C 103/52,** C 12 Q 1/36

(21) Anmeldenummer: **81810036.4**

(22) Anmeldetag: **06.02.81**

(54) **Tripeptidderivate und deren Verwendung zur Bestimmung von Enzymen.**

(30) Priorität: **12.02.80 CH 1130/80**

(43) Veröffentlichungstag der Anmeldung: **19.08.81 Patentblatt 81/33**

(45) Bekanntmachung des Hinweises auf die Patenterteilung: **21.09.83 Patentblatt 83/38**

(84) Benannte Vertragsstaaten: **AT BE CH DE FR GB IT LI LU NL SE**

(56) Entgegenhaltungen:
EP-A-0 004 256
DE-A-2 724 211
FR-A-2 317 278

(73) Patentinhaber: **Pentapharm A.G., Engelgasse 109, CH-4052 Basel (CH)**

(72) Erfinder: **Svendsen, Lars Gundro, Reichensteinerstrasse 15, CH-4153 Reinach/BL (CH)**

**EP 0 034 122 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Tripeptidderivate und deren Verwendung zur Bestimmung von Enzymen

*Technisches Gebiet*

Die vorliegende Erfindung betrifft Tripeptidderivate, die von gewissen Enzymen der Enzymklasse E.C. 3.4.21., insbesondere Faktor Xa, sehr leicht gespalten werden. Die neuen Tripeptidderivate werden deshalb als Substrate zur quantitativen Bestimmung der genannten Enzyme, besonders Faktor Xa, verwendet.

*Zugrundeliegender Stand der Technik*

Der Faktor Xa ist ein proteolytisches Enzym, das in der Blutgerinnungskaskade durch Aktivierung des Proenzyms Faktor X entsteht und zusammen mit Phospholipid und Calciumionen den Faktor II (Prothrombin) an zwei Stellen der Peptidkette proteolytisch spaltet und in Faktor IIa (Thrombin) überführt, welcher letztlich die Gerinnung bewirkt. Bei gewissen pathologischen Zuständen, z.B. bei Leberkrankheiten, Vitaminmangel usw., und bei der Dicumaroltherapie ist die Bildung des Faktors X herabgesetzt. Selbstverständlich ist bei erblichen Störungen der Synthese des Faktors X auch die Bildung des Faktors Xa entsprechend vermindert. Es ist deshalb wichtig, über eine direkte enzymatische Bestimmungsmethode zu verfügen, welche es erlaubt, Faktor Xa auf einfache und genaue Weise photometrisch im Blutplasma zu bestimmen.

Die wichtigsten Methoden zur Bestimmung von Faktor Xa sind die folgenden:

a) *Biologische Bestimmungsmethode* [s. Nils U. Bang, „Thrombosis and Bleeding Disorders", Georg Thieme Verlag, S. 196 (1971)]: Faktor X wird mittels Gift der Russel-Viper und Calciumionen zu Faktor Xa aktiviert. In einer einstufigen Operation wird Prothrombin in Gegenwart von Faktor V und Phospholipiden durch Faktor Xa zu Thrombin aktiviert, welches Indikatorfibrinogen in Fibrin überführt. Es wird die Gerinnungszeit gemessen. Die benötigten Faktoren II und V und fibrinogen werden mittels eines Substrats, das frei von Faktor X ist, zugeführt. Die Gerinnungszeit wird durch den Aktivierungsgrad des Faktors X beeinflusst. Der Aktivierungsgrad ist unter sonst gleichbleibenden Bedingungen eine Funktion der Konzentration an Faktor X in der Probe. Diese biologische Methode erlaubt nur eine grobe Bestimmung, weil die Gerinnungszeit durch den Experimentator subjektiv abgelesen wird. Überdies benötigt man ein manipuliertes Plasma, bei dessen Herstellung Fehler auftreten können. Ferner wird das als Indikator wirkende Fibrinogen nicht direkt, sondern über das aktivierte Thrombin gebildet (indirekte Methode).

b) *Biochemische Methode* [s. Nils U. Bang, „Thrombosis and Bleeding Disorders", Georg Thieme Verlag, S. 196/7 (1971)]: Wenn die zu untersuchenden Faktor-X-Präparate genügend rein sind, kann man eine genauere Bestimmungsmethode anwenden. Esnouf und Williams (1962) haben nachgewiesen, dass Faktor Xa eine Esteraseaktivität aufweist und synthetische Aminosäureester spaltet. Für diese Bestimmung sind jedoch 50 bis 100 µg Faktor X erforderlich. Man kann allerdings noch niedrigere Konzentrationen an Faktor Xa bestimmen, wenn man Carbobenzoxyphenylalanin-p-bitrophenylester verwendet und die pro Zeiteinheit freigesetzte Menge p-Nitrophenol misst. Diese Bestimmungsmethode ist mit den folgenden Nachteilen behaftet: Der verwendete Ester unterliegt bei dem verwendeten pH 8 einer Selbsthydrolyse und ist im übrigen nicht spezifisch für Faktor Xa, da er auch auf viele andere Enzyme anspricht. Der Ester ist in Wasser unlöslich, so dass man gezwungen ist, Aceton zu verwenden. Alle diese Nachteile führen dazu, dass die Bestimmungsmethode ungenau und aufwendig ist.

c) In der deutschen Offenlegungsschrift Nr. 2552570 sind Tetrapeptidderivate beschrieben, die als Substrate zur Bestimmung des Faktors Xa verwendet werden sollen. Als Beispiel ist das Tetrapeptidderivat Bz-Ile-Glu-Gly-Arg-pNA · HCl offenbart, das durch Faktor Xa unter Bildung von p-Nitroanilin gespalten wird. Die Bildung des p-Nitroanilins kann spektrophotometrisch verfolgt werden. Diese Bestimmungsmethode für Faktor Xa ist bereits genauer als die oben beschriebenen biologischen und biochemischen Bestimmungsmethoden.

Die in der DE-OS Nr. 2552570 beschriebenen Tetrapeptidderivate sind in wässerigen Medien nicht genügend löslich, um die Bestimmung des Faktors Xa bei Substratsättigung durchführen zu können. Wenn man extrem niedrige Konzentrationen an Faktor Xa bestimmen muss, z.B. in pathologischem Plasma, so ist die Empfindlichkeit der genannten Tetrapeptidderivate nicht genügend, um vernünftig genaue Messwerte zu erzielen. Wollte man die zu messende Menge an Faktor Xa durch Zugabe von zusätzlichem Plasma erhöhen, so würde unter dem Einfluss der Plasmaproteine eine Ausfällung des Tetrapeptidsubstrates stattfinden, wodurch die Enzymbestimmung verunmöglicht würde.

In der deutschen Auslegeschrift Nr. 2724211, in der französischen Auslegeschrift Nr. 2317278 und in der europäischen Auslegeschrift Nr. 0019589 sind Tripeptidderivate mit abspaltbaren chromogenen Gruppen beschrieben, welche zur Bestimmung von gewissen Enzymen verwendbar, jedoch zur Bestimmung von Faktor Xa ungeeignet sind. In der europäischen Auslegeschrift Nr. 0004256 sind Tripeptidderivate beschrieben, welche entweder eine N-terminale Aminosäure mit L-Form und einer acylierten Aminogruppe oder eine Aminosäure mit D-Form und einer freien Aminogruppe enthalten. Diese Tripeptidderivate, die an sich z.T. zur Bestimmung von Faktor Xa verwendbar sind, besitzen für praktische Zwecke eine ungenügende Empfindlichkeit gegenüber Faktor Xa.

*Offenbarung der Erfindung*

Es wurden nun neue Tripeptidderivate gefunden, die in wässerigen Medien sehr gut löslich sind und gegenüber Faktor Xa eine erstaunlich hohe Empfindlichkeit aufweisen.

Die erfindungsgemässen Tripeptidderivate entsprechen der allgemeinen Formel

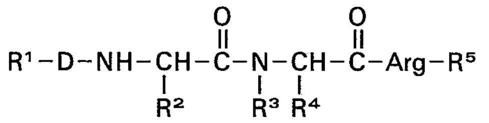

(I)

in welcher

$R^1$ eine gegebenenfalls in ω-Stellung eine Aminogruppe tragende Alkanoylgruppe mit 2 bis 8 Kohlenstoffatomen, eine Phenylalkanoylgruppe, die 2 bis 4 Kohlenstoffatome im Alkanoyl enthält und deren Phenylrest gegebenenfalls in p-Stellung mit einer Aminogruppe substituiert ist, eine gegebenenfalls in 4-Stellung mit einem Aminomethylrest substituierte Cyclohexylcarbonylgruppe, eine gegebenenfalls in o- oder p-Stellung mit Methyl, Amino oder Halogen, z.B. Cl oder Br, substituierte Benzoylgruppe, eine Alkoxycarbonylgruppe mit 1 bis 8 Kohlenstoffatomen in der Alkoxygruppe, eine gegebenenfalls in p-Stellung mit Methoxy, Methyl oder Chlor substituierte Benzyloxycarbonylgruppe, eine Alkansulfonylgruppe mit 1 bis 4 Kohlenstoffatomen, eine gegebenenfalls in p-Stellung methylierte Phenylsulfonylgruppe oder eine α- oder β-Naphthylsulfonylgruppe darstellt,

$R^2$ einen geradkettigen oder verzweigten Alkylrest mit 1 bis 6, vorzugsweise 1 bis 4 Kohlenstoffatomen, einen Hydroxyalkylrest mit 1 bis 2 Kohlenstoffatomen, einen Alkoxyalkylrest mit 1 bis 2 Kohlenstoffatomen im Alkyl und 1 bis 4 Kohlenstoffatomen im Alkoxy, einen Benzyloxyalkylrest mit 1 bis 2 Kohlenstoffatomen im Alkyl, einen ω-Carboxyalkyl- oder ω-Alkoxycarbonylalkylrest mit 1 bis 3 Kohlenstoffatomen im Alkyl, wobei die Alkoxygruppe geradkettig oder verzweigt ist und 1 bis 4 Kohlenstoffatome enthält, oder einen ω-Benzyloxycarbonylalkylrest mit 1 bis 3 Kohlenstoffatomen im Alkyl, oder einen Cyclohexyl-, Cyclohexylmethyl-, 4-Hydroxycyclohexylmethyl-, Phenyl-, Benzyl-, 4-Hydroxybenzyl- oder Imidazolyl-(4)-methylrest darstellt,

$R^3$ Wasserstoff oder einen geradkettigen oder verzweigten Alkylrest mit 1 bis 4 Kohlenstoffatomen darstellt,

$R^4$ Wasserstoff oder einen Methyl- oder Äthylrest darstellt, und

$R^5$ eine mit aromatischen oder heterocyclischen Resten substituierte Aminogruppe, welche hydrolytisch unter Bildung einer farbigen oder fluoreszierenden Verbindung $H-R^5$ abspaltbar ist, darstellt, ausgenommen Tripeptidderivate, in welchen $R^1$ Benzyloxycarbonyl und $R^2$ Isopropyl oder Cyclohexyl bedeuten.

Die stark basische Guanidinogruppe des Arginins ist vorzugsweise stabilisiert, z.B. durch Protonisierung mit einer Mineralsäure, wie HCl, HBr, $H_2SO_4$ oder $H_3PO_4$, oder einer organischen Säure, wie Ameisen-, Essig-, Propion-, Milch-, Zitronen-, Oxal-, Wein-, Benzoe-, Phthal-, Trichloressig- oder Trifluoressigsäure. Die Art der Protonisierung hat auf die Spaltbarkeit, d.h. die Empfindlichkeit (Suszeptibilität), des Tripeptidsubstrates gegenüber den Enzymen überhaupt keinen Einfluss.

Die in der allgemeinen Formel I mit $R^5$ bezeichnete abspaltbare chromogene Gruppe kann z.B. eine p-Nitrophenylamino-, 1-Carboxy-2-nitrophenyl-(5)-amino-, 1-Sulfo-2-nitrophenyl-(5)-amino-, β-Naphthylamino-, 4-Methoxy-β-naphthylamino-, 5-Nitro-α-naphthylamino-, Chinonyl-(5)-amino-, 8-Nitrochinonyl-(5)-amino-, 4-Methylcumaryl-(7)-amino- oder 1,3-Di(methoxycarbonyl)phenyl-(5)-aminogruppe (abgeleitet von 5-Aminoisophthalsäuredimethylester) sein.

Die Tripeptidderivate der Formel I können nach den im folgenden beschriebenen, an sich bekannten Methoden hergestellt werden:

1. Die chromogene Gruppe $R^5$ wird an die Carboxygruppe des C-terminalen Arginins angehängt, wobei dessen α-Aminogruppe durch eine Schutzgruppe, z.B. eine Carbobenzoxy- oder tert.-Butoxycarbonylgruppe, und die δ-Guanidylgruppe des Arginins durch Protonisierung, z.B. mit HCl, Nitrierung oder Tosylierung geschützt werden. Die C-terminale Gruppe $R^5$ dient während des stufenweisen Aufbaus der Peptidkette ebenfalls als Schutzgruppe. Die anderen Schutzgruppen können je nach Bedarf selektiv abgespalten werden, um die weiteren Aminosäurederivate anzuknüpfen, bis die gewünschte Peptidkette vollständig aufgebaut ist. Zum Schluss können die verbleibenden Schutzgruppen vollständig abgespalten werden, ohne dass die Gruppe $R^5$ in Mitleidenschaft gezogen wird [s. z.B. Miklos Bodansky *et al.*, „Peptide Synthesis", Interscience Publishers, S. 163-165 (1966)].

2. Zuerst wird die Peptidkette (nach Bodanksy, *loc. cit.*) aufgebaut, wobei jedoch die C-terminale Carboxylgruppe des Arginins mit einer üblichen Estergruppe, z.B. einer Methoxy-, Äthoxy- oder Benzyloxygruppe, geschützt wird. die Estergruppen können durch alkalische Hydrolyse abgespalten werden, mit Ausnahme der tert.-Butoxygruppe, die selektiv mittels Trifluoressigsäure abgespalten werden muss. Falls die δ-Guanidylgruppe des Arginins protonisiert ist, wird die genannte Estergruppe mittels Trypsin abgespalten, wobei keine Racemisierung eintritt. Hierauf wird die chromogene Gruppe $R^5$ angeknüpft. Wenn die δ-Guanidinogruppe des Arginins durch eine Nitro- oder Tosylgruppe und die N-terminale α-Aminogruppe des Tripeptidderivates durch eine Carbobenzoxygruppe oder eine p-Methyl-, p-Methoxy- oder p-Chlorbenzyloxycarbonylgruppe oder eine tert.-Butoxygruppe geschützt sind, so werden diese Schutzgruppen gleichzeitig abgespalten. Die Abspaltung kann durch Behandlung des geschützten Tripeptidderivats mit wasserfreiem HF bei Raumtemperatur durchgeführt werden, wobei alle oben genannten Amino- bzw. δ-Guanidinoschutzgruppen abgespalten werden. Die Abspaltung kann auch durch Behandlung mit 2N HBr

in Eisessig bei Raumtemperatur durchgeführt werden, wenn das geschützte Tripeptidderivat keine Nitro- oder Tosylschutzgruppe enthält.

Die Tripeptidderivate der Formel I sind in Wasser wesentlich leichter löslich als die in der DE-OS Nr. 2552570 beschriebenen Tetrapeptidderivate, so dass mit diesen Tripeptidderivaten die Enzymbestimmungen bei der zur Erzielung zuverlässiger Messresultate erforderlichen Substratsättigung durchgeführt werden können. Die Tripeptidsubstrate der Formel I sind ferner signifikant empfindlicher als die in der DE-OS Nr. 2552570 beschriebenen Tetrapeptidderivate und können deshalb auch zur Bestimmung extrem kleiner Konzentrationen an Faktor Xa verwendet werden.

In der niederländischen OS Nr. 7607433 sind Tripeptidderivate der allgemeinen Formel $H-D-A_1-A_2-A_3-NH-R(A_1, A_2, A_3$ = Aminosäurereste) beschrieben, die eine hohe Empfindlichkeit gegenüber Enzymen der Enzymklasse E.C. 3.4.21., z.B. Thrombin und Plasmin, besitzen. Diese hohe Empfindlichkeit wird darauf zurückgeführt, das die N-terminale D-Aminosäure an der α-Aminogruppe unsubstituiert ist. Wird nämlich die α-Aminogruppe der N-terminalen D-Aminosäure substituiert, d.h. der Wasserstoff in der Formel durch eine Schutz- oder Blockierungsgruppe, z.B. durch Benzoyl oder Carbobenzoxy, ersetzt, so sinkt die Empfindlichkeit des Tripeptidderivates gegenüber Enzymen der Enzymklasse E.C. 3.4.21. in drastischem Ausmass. Auf Grund dieses Befundes hätte man erwarten können, dass die neuen Tripeptidderivate der Formel I, in welchen die α-Aminogruppe der N-terminalen D-Aminosäure eine Schutz- oder Blockierungsgruppe trägt, von Enzymen der Enzymklasse E.C. 3.4.21. nicht oder nur geringfügig gespalten würden. Ganz im Gegenteil weisen die neuen Tripeptidsubstrate gegenüber Faktor Xa unerwarteterweise eine sehr hohe Empfindlichkeit auf.

Ein weiterer Vorteil, den die erfindungsgemässen Tripeptidsubstrate gegenüber den in der DE-OS Nr. 2552570 beschriebenen Tetrapeptidderivaten besitzen, besteht darin, dass deren Synthese einfacher und weniger kostspielig ist.

Die erfindungsgemässen Tripeptidsubstrate können zur quantitativen Bestimmung von gewissen Enzymen der Enzymklasse E.C. 3.4.21. [s. „Enzyme Nomenclature", Elsevier Scientific Publishing Company, Amsterdam (1973), S. 238 f.], z.B. von Faktor Xa in Blutplasma und über die Bestimmung von Faktor Xa auch zur quantitativen Bestimmung von anderen biologisch wichtigen Faktoren, z.B. Faktor X (Proenzym des Faktors Xa), Antifaktor Xa (= Antithrombin III oder Heparincofaktor) oder Heparin in Blutplasma, verwendet werden.

Diese verschiedenen Bestimmungen können in der nachfolgend beschriebenen Weise durchgeführt werden:

*1) Bestimmung von Faktor Xa und Faktor X:*

Man verwendet menschliches Zitratplasma, das man zuerst mit Gift der Russel-Viper (RVV = Russel viper venom) aktiviert, um den im Plasma enthaltenen Faktor X in Faktor Xa überzuführen. Man verwendet die folgenden Reagenzien:

— Puffer: Trisimidazolpuffer, pH 8,4, Ionenstärke 0,3;

— RVV: gefriergetrocknetes Russel-Viper-Giftpräparat der Firma Laboratoire Stago, 92600 Asnières s/Seine, Frankreich; gelöst in 2 ml Puffer, der $CaCl_2$ in 0,015-molarer Konzentration enthält;

— Substrat: Cbo-D-Leu-Sar-Arg-pNA · AcOH, gelöst in dest. $H_2O$, Konzentration: $2 \times 10^{-3}$-molar.

In eine Testküvette werden 0,2 ml auf 37°C erwärmtes RVV-Reagenz und dann 0,02 ml menschliches Zitratplasma gegeben. Die beiden Komponenten werden sofort gemischt und dann während 60 s bei 37°C inkubiert. Das erhaltene Aktivierungsgemisch wird mit 1,6 ml Puffer von 37°C verdünnt und mit 0,2 ml $2 \times 10^{-3}$-molarer Substratlösung gemischt. Hierauf misst man spektrophotometrisch die durch das freigesetzte p-Nitroanilin bewirkte Änderung der optischen Dichte des Gemisches bei einer Wellenlänge von 405 nm. Die gemessene Zunahme der optischen Dichte pro Minute ist der im aktivierten Plasma vorhandenen Menge an Faktor Xa direkt proportional. Die gemessene Zunahme der optischen Dichte in 1 min ist zugleich ein Mass für die im Ausgangsplasma vorhandene Menge an Faktor X, da bei der Aktivierung eine gegebene Menge Faktor X stöchiometrisch in die entsprechende Menge Faktor Xa umgewandelt wird.

*2) Bestimmung von Antifaktor Xa:*

Man stellt eine Testprobe dar, indem man menschliches Zitratplasma mit Trisimidazolpuffer im Verhältnis von 1:10 verdünnt. Man gibt 100 µl einer Heparinlösung in Trisimidazolpuffer (enthaltend 3 USP-Einheiten Heparin pro Milliliter Puffer) zu 100 µl des verdünnten Plasmas und inkubiert die Mischung während 2 min bei 37°C. Man setzt dem Inkubat 100 µl einer 8,5 Einheiten Faktor Xa pro Milliliter enthaltenden Lösung des Faktor-Xa-Präparates Diagen (Lieferant: Diagnostic Reagents Ltd., Thame, Grossbritannien) zu. Die Mischung wird während 3 min bei 37°C inkubiert. Das Inkubat wird mit 0,6 ml Trisimidazolpuffer von 37°C verdünnt und sofort mit 100 µl einer auf 37°C erwärmten $2 \times 10^{-3}$-molaren Lösung von Cbo-D-Leu-Sar-Arg-pNA · AcOH (Substrat) in dest. Wasser gemischt.

In einem Parallelversuch wird eine Blindprobe in der gleichen Weise hergestellt, indem jedoch anstelle des verdünnten Plasmas die gleiche Volumenmenge Puffer verwendet wird.

Für beide Proben (Testprobe und Blindprobe) wird die Zunahme der optischen Dichte bei 405 nm spektrophotometrisch gemessen. Die Differenz zwischen der Zunahme der optischen Dichte der Blindprobe pro Minute ($\Delta OD_{Blind}$/min) und der Zunahme der optischen Dichte der Testprobe pro Minute ($\Delta OD_{Test}$/min) ist ein Mass für die Menge des durch den Antifaktor-Xa/Heparin-Komplex gehemmten Faktors Xa und entsprechend ein Mass für die im Plasma vorhandene Menge Antifaktor Xa (= Antithrombin III).

*3) Bestimmung von Heparin in Blutplasma:*

Man stellt eine Testprobe dar, indem man Plasma eines mit Heparin behandelten Patienten mit Trisimidazolpuffer im Verhältnis von 1:10 verdünnt. Das verdünnte Plasma wird während 1 bis 2 min bei 37°C inkubiert. Zu 200 µl des Inkubats gibt man 100 µl einer 8,5 Einheiten Faktor Xa pro Milliliter enthaltenden Lösung des Faktor-Xa-Präparates Diagen (Lieferant: Diagnostic Reagents Ltd., Thame, Grossbritannien) und inkubiert die Mischung während 3 min bei 37°C. Das Inkubat wird mit 0,6 ml Trisimidazolpuffer von 37°C verdünnt und sofort mit 100 µl einer $2 \times 10^{-3}$-molaren Lösung von Cbo-D-Leu-Sar-Arg-pNA · AcOH (Substrat) in dest. Wasser gemischt.

In einem Parallelversuch wird eine Blindprobe in der gleichen Weise hergestellt, indem jedoch anstelle des verdünnten heparinhaltigen Plasmas die gleiche Menge von entsprechend verdünntem Normalplasma (heparinfrei) verwendet wird.

Für beide Proben wird die Zunahme der optischen Dichte pro Minute bei 405 nm spektrophotometrisch gemessen. Die Differenz zwischen der Zunahme der optischen Dichte der Blindprobe pro Minute ($\Delta OD_{Blind}$/min) und der Zunahme der optischen Dichte der Testprobe pro Minute ($\Delta OD_{Test}$/min) ist ein Mass für die Hemmwirkung des an Heparincofaktor (Antithrombin III) gebundenen Heparins.

Antifaktor Xa (= Heparincofaktor oder Antithrombin III) allein hemmt Faktor Xa nur langsam, so dass bei einer kurzen Inkubationszeit (z.B. 3 min) nur geringe Mengen Faktor Xa gehemmt werden. Wenn aber Antifaktor Xa mit Hearin in Verbindung tritt, so bildet sich zwischen den beiden Komponenten ein Komplex, der ein schneller Hemmer für Faktor Xa ist und innerhalb einer kurzen Inkubationszeit (z.B. 3 min) sich vollständig mit Faktor Xa verbindet.

Da die Hemmung des Faktors Xa der Menge des vorhandenen Heparins proportional ist, solange genügend Antifaktor Xa vorhanden ist, kann man anhand einer Eichkurve, die mit Normalplasma und steigenden Mengen Heparin aufgestellt wird, auf Grund der potenzierenden Wirkung des zugesetzten Heparins die Menge des im Patientenplasma vorhandenen Heparins ermitteln.

Bei der Messung der bei der Umsetzung des Faktors Xa mit den erfindungsgemässen Tripeptidderivaten (Substraten) freigesetzten Menge des farbigen Spaltproduktes $H-R^5$ (p-Nitroanilin) wird von der Tatsache Gebrauch gemacht, dass das Spaltprodukt ein UV-Spektrum aufweist, welches von demjenigen des Substrats verschieden und gegen höhere Wellenlängen verschoben ist. Die erfindungsgemässen Substrate besitzen ein Absorptionsmaximum bei etwa 310 nm und einen molaren Extinktionskoeffizienten von etwa 13000. Die Absorption des Substrates ist bei 405 nm praktisch Null. Das durch die enzymatische Hydrolyse des Substrates gebildete Spaltprodukt $H-R^5$, d.h. p-Nitroanilin, weist ein Absorptionsmaximum bei 380 nm und einen molaren Extinktionskoeffizienten von etwa 13200 auf. Bei 405 nm ist der Extinktionskoeffizient nur mässig, d.h. auf etwa 10400, reduziert.

Für Substrate, die als chromogene Gruppe eine β-Naphthylamino-, 4-Methoxy-β-naphthylamino-, Cumaryl-(7)-amino- oder Isophthalylaminogruppe enthalten, wird die Menge des durch den Faktor Xa abgespaltenen Produktes $H-R^5$ durch Fluoreszenzspektrophotometrie gemessen. In einem aus Faktor Xa, Puffer und Substrat bestehenden Testsystem misst man kontinuierlich das energieärmere emittierte Licht bei 400 bis 470 nm, nachdem man das fluoreszierende Spaltprodukt mit energiereicherem Licht von 300 bis 400 nm kontinuierlich angeregt hat. Die pro Zeiteinheit gebildete Menge des Spaltproduktes ist ein Mass für die vorhandene Faktor-Xa-Aktivität. 1 µmol des pro Minute gebildeten Spaltproduktes entspricht definitionsgemäss 1 Enzymeinheit Faktor Xa, bezogen auf ein gegebenes Substrat.

Die Empfindlichkeit der oben beschriebenen Bestimmungsmethoden kann noch erhöht werden, indem man das Spaltprodukt $H-R^5$, wenn $R^5$ eine p-Nitrophenylamino-, 1-Carboxy-2-nitrophenyl-(5)-amino-, 1-Sulfo-2-nitrophenyl-(5)-amino-, 5-Nitro-α-naphthylamino- oder 8-Nitrochinonyl-(5)-aminogruppe ist, vor der Messung durch Kupplung mit einer Diazoverbindung in eine intensiver gefärbte Verbindung überführt.

*Bester Weg zur Durchführung der Erfindung*

In den nachfolgenden Ausführungsbeispielen ist die Herstellung der erfindungsgemässen Tripeptidderivate ausführlicher beschrieben. Temperaturen sind in Celsiusgraden angegeben.

Die Analysen der gemäss den Beispielen erhaltenen Eluate und Produkte wurden durch Dünnschichtchromatographie unter Verwendung von mit Siliciumdioxydgel überzogenen Glasplatten (Merck, F 254) durchgeführt. Die Dünnschichtchromatogramme wurden mittels des folgenden Lösungsmittelsystems entwickelt: n-Butanol/Essigsäure/Wasser (3:1:1).

Es werden die folgenden Abkürzungen verwendet:

D-Aadi = D-α-Aminoadipinsäure
Ac = Acetyl
$Ac_2O$ = Acetanhydrid
AcOH = Essigsäure
Ala = L-Alanin
D-Ala = D-Alanin
AOA = D-α-Aminooctansäure
Arg = L-Arginin
D-Asp = D-Asparaginsäure
BOC = tert.-Butoxycarbonyl
Bu = Butyl
But = L-2-Aminobuttersäure
D-But = D-2-Aminobuttersäure
Bz = Benzoyl
Bzl = Benzyl
$Bz_2O$ = Benzoesäureanhydrid
ChA = Chinonylamid
D-CHA = D-3-Cyclohexylalanin
D-CHG = D-2-Cyclohexylglycin
D-CHT = D-3-(4-Hydroxycyclohexyl)alanin = kernhydriertes Tyrosin

Cbo = Carbobenzoxy
DMF = Dimethylformamid
DPA = 5-Aminoisophthalsäuredimethylester
DSC = Dünnschichtchromatogramm bzw. Dünnschichtchromatographie
Et = Äthyl
$Et_3N$ = Triäthylamin
Gly = Glycin
D-Glu = D-Glutaminsäure
D-His = D-Histidin
HMPTA = N,N,N',N',N",N"-Hexylmethylphosphorsäuretriamid
D-Ile = D-Isoleucin
D-Leu = D-Leucin
LMS = Lösungsmittelsystem
MCA = 7-Amino-4-methylcumarin
MeO = Methoxy
MeOH = Methanol
Na = Naphthylamin
D-Nleu = D-Norleucin
D-Nval = D-Norvalin
OtBu = tert.-Butoxy
OpNP = p-Nitrophenoxy
pNA = p-Nitroanilid
Pr = Propyl
D-Ph'Gly = D-2-Phenylglycin
D-Phe = D-Phenylalanin
Sar = Sarkosin = N-Methylglycin
D-Ser = D-Serin
TFA = Trifluoressigsäure
THF = Tetrahydrofuran
D-Thr = D-Threonin
Tos = p-Toluolsulfonyl
D-Tyr = D-Tyrosin
D-Val = D-Valin

Wenn nichts anderes vermerkt ist, weisen die Aminosäuren die L-Form auf.

*Beispiel 1:*

*Cbo-D-Leu-Gly-Arg-pNA· HBr*

1a) *Cbo-Arg-pNA· HCl*

In einem Dreihalsrundkolben von 250 ml Inhalt wurden 16,0 g (47,0 mmol) über $P_2O_5$ im Vakuum getrocknetes Cbo-Arg-OH·HCl in 90 ml abs. HMPTA unter Feuchtigkeitsausschluss bei 20° gelöst. Bei Zimmertemperatur wurden der erhaltenen Lösung zuerst eine Lösung von 4,74 g (47,0 mmol) $Et_3N$ in 10 ml HMPTA und dann 16,4 g (100 mmol) p-Nitrophenylisocyanat (100%iger Überschuss) portionenweise zugesetzt. Nach 24 h Reaktionszeit bei 20° wurde das HMPTA im Vakuum grösstenteils abdestilliert. Der Rückstand wurde mehrmals mit 30%iger AcOH extrahiert. Der Rückstand wurde verworfen. Die vereinigten AcOH-Extrakte wurden zur weiteren Reinigung auf eine mit 30%iger AcOH äquilibrierte Sephadex-G-15-Säule aufgetragen und mit 30%iger AcOH eluiert. Diejenige Fraktion des AcOH-Eluates, die sich durch Trypsinbehandlung unter Freisetzung von p-Nitroanilin spalten liess, wurde gefriergetrocknet. Man erhielt 12,6 g eines amorphen Pulvers, das im DSC im LMS einheitlich war.

*Elementaranalyse* und Berechnung aus der Bruttoformel $C_{20}H_{25}N_6O_5Cl$ ergaben die folgenden Werte:

| | | | | |
|---|---|---|---|---|
| gef.: | C 51,29 | H 5,48 | N 17,92 | Cl 7,50% |
| ber.: | C 51,67 | H 5,42 | N 18,08 | Cl 7,63% |

1b) *2HBr· H-Arg-pNA*

Unter Feuchtigkeitsausschluss wurden 4,65 g (10 mmol) der Verbindung 1a mit 40 ml 2N HBr in Eisessig unter Rühren 45 min bei 20° behandelt. Das Aminosäurederivat löste sich dabei unter $CO_2$-Entwicklung. Die Reaktionslösung wurde unter intensivem Rühren zu 250 ml abs. Äther zugetropft, wobei 2HBr·H-Arg-pNA ausfiel. Die Ätherphase wurde abgesaugt, worauf die feste Phase noch viermal mit je 100 ml abs. Äther gewaschen wurde, um das als Nebenprodukt gebildete Benzylbromid sowie den Überschuss an HBr und AcOH weitgehend zu entfernen. Der Rückstand wurde in 50 ml MeOH gelöst, mit $Et_3N$ auf pH 4,5 eingestellt und zur Trocknung im Vakuum bei 30° eingeengt. Dieses so erhaltene Produkt in 75 ml MeOH gelöst und durch eine mit MeOH äquilibrierte Säule von Sephadex LH-20 (vernetztes Dextrangel) laufen gelassen. Aus einer Fraktion des Eluates erhielt man 4,18 g (91,6% der Theorie) der amorphen Verbindung 1b, die im DSC im LMS einheitlich war.

*Elementaranalyse* und Berechnung aus der Bruttoformel $C_{12}H_{20}N_6O_3Br_2$ ergaben die folgenden Werte:

| | | | | |
|---|---|---|---|---|
| gef.: | C 31,15 | H 4,35 | N 18,84 | Br 34,81% |
| ber.: | C 31,60 | H 4,42 | N 18,43 | Br 35,03% |

1c) *Cbo-Gly-Arg-pNA· HBr*

4,56 g (10 mmol) der Verbindung 1b wurden in 30 ml frisch destilliertem DMF gelöst und nach Kühlung auf −10° unter Rühren mit 1,40 ml (10 mmol) $Et_3N$ versetzt. Das gebildete $Et_3N$·HBr wurde abfiltriert und mit wenig kaltem DMF gewaschen. Man gab dem Filtrat unter Rühren bei −10° 3,65 g (11 mmol) Cbo-Gly-OpNP zu und liess das Gemisch unter Feuchtigkeitsausschluss 2 bis 3 h lang reagieren, wobei die Temperatur der Reaktionslösung allmählich auf etwa 20° stieg. Die Lösung wurde wieder auf −10° gekühlt und mit 0,70 ml (5 mmol) $Et_3N$ gepuffert. Man liess die Reaktionslösung etwa 2 h bei −10° und 3 h bei Raumtemperatur reagieren. Diese Prozedur wurde nochmals mit 0,70 ml $Et_3N$ wiederholt, und nach weiteren 16 h wurde die Reaktionslösung im Vakuum bei 50° zur Trockne eingeengt. Der Rückstand wurde in 75 ml 50%iger AcOH gelöst und durch Gelfiltration auf einer mit 50%iger AcOH äquilibrierten Säule von Sephadex G-15 gereinigt. Diejenige Fraktion des AcOH-Eluates, die sich durch Trypsinbehandlung unter Freisetzung von p-Nitroanilin spalten liess, wurde im Vakuum bei 40° zur Trockne eingeengt. Der Rückstand wurde in 150 ml MeOH gelöst und nochmals zur Trockne eingeengt. Nach Trocknung des erhaltenen Rückstandes im Vakuumtrockenschrank bei 60° über $P_2O_5$ erhielt man 5,85 g (88,3% der Theorie) der amorphen Verbindung 1c, die im DSC im LMS einheitlich war.

*Elementaranalyse* und Berechnung aus der Bruttoformel $C_{22}H_{28}N_7O_6Br$ ergaben die folgenden Werte:

| | | | | |
|---|---|---|---|---|
| gef.: | C 46,33 | H 5,04 | N 17,88 | Br 14,20% |
| ber.: | C 46,65 | H 4,98 | N 17,31 | Br 14,11% |

1d) *2HBr· H-Gly-Arg-pNA*

4,56 g (8 mmol) der Verbindung 1c wurden unter Feuchtigkeitsausschluss mit 32 ml 2N HBr in Eisessig unter Rühren 40 min lang bei 20° behandelt. Das Dipeptidderivat löste sich dabei allmählich unter $CO_2$-Entwicklung. Die Reaktionslösung wurde unter intensivem Rühren zu 250 ml abs. Äther zugetropft, wobei 2HBr · H-Gly-Arg-pNA ausfiel. Die Ätherphase wurde abgesaugt, worauf die feste Phase noch viermal mit je 100 ml abs. Äther gewaschen wurde, um das als Nebenprodukt gebildete Benzylbromid sowie den Überschuss an HBr und AcOH weitgehend zu entfernen. Der Rückstand wurde in 50 ml MeOH gelöst. Nach Einstellung des pH auf 4,5 mit $Et_3N$ wurde die Lösung im Vakuum bei 30° zur Trockne eingeengt. Der so erhaltene Rückstand wurde in 50 ml MeOH gelöst und auf einer mit MeOH äquilibrierten Säule von Sephadex LH-20 gereinigt. Diejenige Fraktion des MeOH-Eluates, die sich durch Trypsinbehandlung unter Freisetzung von p-Nitroanilin spalten liess, wurde im Vakuum bei 30° zur Trockne eingeengt. Nach Trocknung des erhaltenen Rückstandes im Vakuumtrockenschrank bei 40° über $P_2O_5$ erhielt man 3,78 g (92,1% der Theorie) der amorphen Verbindung 1d, die im DSC im LMS einheitlich war.

*Elementaranalyse* und Berechnung aus der Bruttoformel $C_{14}H_{23}N_7O_4Br_2$ ergaben die folgenden Werte:

| | | | | |
|---|---|---|---|---|
| gef.: | C 32,31 | H 4,59 | N 19,47 | Br 30,78% |
| ber.: | C 32,77 | H 4,52 | N 19,11 | Br 31,14% |

1e) *Cbo-D-Leu-Gly-Arg-pNA· HBr*

2,57 g (5 mmol) der Verbindung 1d wurden in 20 ml frisch destilliertem DMF gelöst und nach Kühlung auf −10° unter Rühren mit 0,70 ml (5 mmol) $Et_3N$ versetzt. Das gebildete $Et_3N$ · HBr wurde abfiltriert und mit wenig kaltem DMF nachgewaschen. Zum Filtrat wurden unter Rühren bei −10° 2,13 g (5,5 mmol) Cbo-D-Leu-OpNP gegeben. Man liess das Reaktionsgemisch unter Feuchtigkeitsausschluss 2 bis 3 h lang reagieren, worauf die Temperaturen der Reaktionslösung allmählich auf etwa 20° stieg. Die Lösung wurde wieder auf −10° gekühlt und mit 0,35 ml (2,5 mmol) $Et_3N$ gepuffert. Man liess die Reaktionslösung etwa 2 h bei −20° und weitere 3 h bei Raumtemperatur reagieren. Diese Prozedur wurde nochmals mit 0,35 ml $Et_3N$ wiederholt, und nach weiteren 16 h wurde die Reaktionslösung im Vakuum bei 50° zur Trockne eingeengt. Der Rückstand wurde in 50 ml 50%iger AcOH gelöst und durch Gelfiltrierung auf einer mit 50%iger AcOH äquilibrierten Säule von Sephadex G-15 gereinigt. Diejenige Fraktion des AcOH-Eluates, die sich durch Trypsinbehandlung unter Freisetzung von p-Nitroanilin spalten liess, wurde im Vakuum bei 40° zur Trockne eingeengt. Der Rückstand wurde in 100 ml MeOH gelöst, worauf die Lösung nochmals zur Trockne eingeengt wurde. Nach Trocknung des erhaltenen Rückstandes im Vakuumtrockenschrank bei 60° über $P_2O_5$ erhielt man 3,08 g (90,6% der Theorie) der amorphen Verbindung 1e, die im DSC im LMS einheitlich war.

*Elementaranalyse* und Berechnung aus der Bruttoformel $C_{28}H_{39}N_8O_7Br$ ergaben die folgenden Werte:

| | | | | |
|---|---|---|---|---|
| gef.: | C 49,06 | H 5,82 | N 16,85 | Br 11,59% |
| ber.: | C 49,49 | H 5,78 | N 16,49 | Br 11,76% |

Die Aminosäureanalyse ergab die zu erwartenden Aminosäuren im richtigen Verhältnis: Gly: 1,00; D-Leu: 0,99; Arg: 0,97.

*Beispiel 2:*

*Cbo-D-Leu-Gly-Arg-MCA· HBr*

2b) *2HBr· H-Arg-MCA*

13,0 g (25,9 mmol) käufliches Cbo-Arg-MCA · HCl wurden mit 104 ml (208 mmol) einer Lösung von 2N HBr in Eisessig gemäss Beispiel 1b deblockiert. Der trockene Rückstand wurde in 400 ml MeOH gelöst und auf einer Säule von Sephadex LH-20 gereinigt. Diejenige Fraktion des MeOH-Eluates, die sich durch Trypsinbehandlung unter Freisetzung von 4-Methyl-7-aminocumarin spalten liess, wurde im Vakuum bei 30° zur Trockne eingeengt. Nach Trocknung des erhaltenen Rückstandes im Vakuumtrockenschrank bei 40° über $P_2O_5$ erhielt man 11,2 g (87,7% der Theorie) der amorphen Verbindung 2b, die im DSC im LMS einheitlich war.

*Elementaranalyse* und Berechnung aus der Bruttoformel $C_{16}H_{23}N_5O_3Br_2$ ergaben die folgenden Werte:

| | | | | |
|---|---|---|---|---|
| gef.: | C 39,40 | H 4,61 | N 14,48 | Br 31,90% |
| ber.: | C 38,96 | H 4,70 | N 14,20 | Br 32,40% |

2c) *Cbo-Gly-Arg-MCA· HBr*

4,93 g (10 mmol) der Verbindung 2b und 3,65 g (11 mmol) Cbo-Gly-OpNP wurden zu 75 ml frisch destilliertem DMF gegeben. Nach Kühlung auf −10° wurden unter Rühren zuerst 1,40 ml (10 mmol) und anschliessend 0,70 ml (5 mmol) $Et_3N$ zugegeben. Man liess das Gemisch unter Feuchtigkeitsausschluss zuerst 3 h bei −10° und dann weitere 4 h bei Raumtemperatur reagieren. Die Reaktionslösung wurde erneut auf −10° gekühlt, mit 0,70 ml $Et_3N$ gepuffert und über Nacht bei 20° gerührt. Das Reaktionsgemisch wurde im Vakuum bei 50° zur Trockne eingeengt, worauf der Rückstand in 200 ml 50%iger AcOH gelöst und auf einer Säule von Sephadex G-15 gereinigt wurde. Diejenige Fraktion des AcOH-Eluates, die sich durch Trypsinbehandlung unter Freisetzung von 4-Methyl-7-aminocumarin spalten liess, wurde im Vakuum bei 40° zur Trockne eingeengt. Nach Trocknung des erhaltenen Rückstandes im Vakuumtrockenschrank bei 60° über $P_2O_5$ erhielt man 4,98 g (82,5% der Theorie) der amorphen Verbindung 2c, die im DSC im LMS einheitlich war.

*Elementaranalyse* und Berechnung aus der Bruttoformel $C_{28}H_{31}N_6O_6Br$ ergaben die folgenden Werte:

| | | | | |
|---|---|---|---|---|
| gef.: | C 51,48 | H 5,24 | N 13,70 | Br 13,14% |
| ber.: | C 51,75 | H 5,18 | N 13,93 | Br 13,24% |

2d) *2HBr· H-Gly-Arg-MCA*

4,83 g (8 mmol) der Verbindung 2c wurden gemäss Beispiel 1d mit 32 ml 2N HBr in Eisessig deblockiert. Das erhaltene Rohprodukt wurde in 100 ml MeOH gelöst und auf einer Säule von Sephadex LH-20 gereinigt. Diejenige Fraktion des MeOH-Eluates, die sich durch Trypsinbehandlung unter Freisetzung von 4-Methyl-7-aminocumarin spalten liess, wurde im Vakuum bei 30° zur Trockne eingeengt. Nach Trocknung des erhaltenen Rückstandes im Vakuumtrockenschrank bei 40° über $P_2O_5$ erhielt man 4,05 g (92,0% der Theorie) der amorphen Verbindung 2d, die im DSC im LMS einheitlich war.

*Elementaranalyse* und Berechnung aus der Bruttoformel $C_{18}H_{26}N_6O_4Br_2$ ergaben die folgenden Werte:

| | | | | |
|---|---|---|---|---|
| gef.: | C 39,02 | H 4,78 | N 15,39 | Br 28,72% |
| ber.: | C 39,29 | H 4,76 | N 15,27 | Br 29,04% |

2e) *Cbo-D-Leu-Gly-Arg-MCA· HBr*

2,75 g (5 mmol) der Verbindung 2d wurden gemäss Beispiel 1e mit 2,13 g (5,5 mmol) Cbo-D-Leu-OpNP umgesetzt. Das erhaltene Rohrprodukt wurde in 75 ml 50%iger AcOH gelöst und auf einer Säule von Sephadex G-15 gereinigt. Diejenige Fraktion des AcOH-Eluates, die sich durch Trypsinbehandlung unter Freisetzung von 4-Methyl-7-aminocumarin spalten liess, wurde im Vakuum bei 40° zur Trockne eingeengt. Nach Trocknung des Rückstandes im Vakuumtrockenschrank bei 60° über $P_2O_5$ erhielt man 2,91 g (81,2% der Theorie) der amorphen Verbindung 2e, die im DSC im LMS einheitlich war.

*Elementaranalyse* und Berechnung aus der Bruttoformel $C_{32}H_{42}N_7O_7Br$ ergaben die folgenden Werte:

| | | | | |
|---|---|---|---|---|
| gef.: | C 53,13 | H 6,01 | N 13,91 | Br 10,88% |
| ber.: | C 53,63 | H 5,91 | N 13,68 | Br 11,15% |

Die Aminosäureanalyse ergab die zu erwartenden Aminosäuren im richtigen Verhältnis:
Gly: 1,00; Leu: 1,02; Arg: 0,98.

*Beispiel 3:*

*Cbo-D-Leu-Gly-Arg-DPA· HBr*

3a) *Cbo-Arg-DPA· HCl*

In einem Dreihalsrundkolben von 1000 ml Inhalt wurden 34,48 g (0,1 mol) getrocknetes Cbo-Arg-OH · HCl in einem Gemisch von 150 ml frisch destilliertem wasserfreiem DMF und 300 ml abs. THF bei 20° gelöst. Der auf −10° gekühlten Lösung wurden unter Rühren und Feuchtigkeitsausschluss 10,2 g (0,1 mol) $Et_3N$ zugesetzt. Dann wurde dem Gemisch innerhalb von 20 min eine Lösung von 13,65 g (0,1 mol) Chlorameisensäureisobutylester in 50 ml THF tropfenweise zugesetzt, wobei man die Reaktionstemperatur nie über −5° steigen liess. Nach einer zusätzlichen Reaktionszeit von 10 min bei einer Temperatur von −10 bis −5° wurde dem Reaktionsgemisch eine Lösung von 20,92 g (0,1 mol) 5-Aminoisophthalsäuredimethylester in 75 ml DMF innerhalb von 30 min tropfenweise zugesetzt, wobei man die Reaktionstemperatur immer unterhalb −5° hielt. Man liess das Reaktionsgemisch noch 1 h bei −5° weiterreagieren. Dann wurde es über Nacht bei 20° gerührt und dann auf −15° gekühlt, um das $Et_3N \cdot HCl$ auskristallisieren zu lassen. Das gebildete $Et_3N \cdot HCl$ wurde abfiltriert und mit wenig kaltem DMF nachgewaschen. Das Filtrat wurde zusammen mit der Waschlösung im Vakuum bei 50° zur Trockne eingeengt. Der Rückstand wurde in 1000 ml 50%iger AcOH gelöst und durch Gelfiltrierung auf einer mit 50%iger AcOH äquilibrierten Säule von Sephadex G-15 gereinigt. Diejenige Fraktion des AcOH-Eluates, die sich durch Trypsinbehandlung unter Freisetzung von 5-Aminoisophthalsäuredimethylester spalten liess, wurde im Vakuum bei 40° zur Trockne eingeengt. Nach Trocknung des Rückstandes im Vakuumtrockenschrank bei 50° über $P_2O_5$ erhielt man 24,6 g (45,9% der Theorie) der amorphen Verbindung 3a, die im DSC im LMS einheitlich war.

*Elementaranalyse* und Berechnung aus der Bruttoformel $C_{24}H_{30}N_5O_7Cl$ ergaben die folgenden Werte:

| | | | | |
|---|---|---|---|---|
| gef.: | C 53,21 | H 5,71 | N 13,20 | Cl 6,52% |
| ber.: | C 53,78 | H 5,64 | N 13,07 | Cl 6,62% |

3b) *2HBr· H-Arg-DPA*

21,44 g (40 mmol) der Verbindung 3a wurden gemäss Beispiel 1b deblockiert. Nach Aufarbeitung wurde das erhaltene Rohprodukt in 250 ml MeOH gelöst und durch Gelfiltrierung auf einer Säule von Sephadex LH-20 gereinigt. Diejenige Fraktion des MeOH-Eluates, die sich durch Trypsinbehandlung unter Freisetzung von 5-Aminoisophthalsäuredimethylester spalten liess, wurde im Vakuum zur Trockne eingeengt. Nach Trocknung des Rückstandes im Vakuumtrockenschrank bei 40° über $P_2O_5$ erhielt man 19,63 g (93,1% der Theorie) der amorphen Verbindung 3b, die im DSC im LMS einheitlich war.

*Elementaranalyse* und Berechnung aus der Bruttoformel $C_{16}H_{25}N_5O_5Br_2$ ergaben die folgenden Werte:

| | | | | |
|---|---|---|---|---|
| gef.: | C 36,82 | H 4,67 | N 13,45 | Br 29,85% |
| ber.: | C 36,45 | H 4,78 | N 13,28 | Br 30,31% |

3c) *Cbo-Gly-Arg-DPA· HBr*

5,27 g (10 mmol) der Verbindung 3b wurden gemäss Beispiel 1c mit 3,65 g (11 mmol) Cbo-Gly-OpNP umgesetzt. Das nach Aufarbeitung erhaltene Rohprodukt wurde in 200 ml 50%iger AcOH gelöst und auf einer Säule von Sephadex G-15 gereinigt. Diejenige Fraktion des AcOH-Eluates, die sich durch Trypsinbehandlung unter Freisetzung von 5-Aminoisophthalsäuredimethylester spalten liess, wurde im Vakuum bei 40° zur Trockne eingeengt. Nach Trocknung des Rückstandes im Vakuumtrockenschrank bei 60° über $P_2O_5$ er-

hielt man 5,29 g (83,0% der Theorie) der amorphen Verbindung 3c, die im DSC im LMS einheitlich war.

*Elementaranalyse* und Berechnung aus der Bruttoformel $C_{26}H_{33}N_6O_8Br$ ergaben die folgenden Werte:

| | | | | |
|---|---|---|---|---|
| gef.: | C 48,50 | H 5,28 | N 12,92 | Br 12,33% |
| ber.: | C 48,99 | H 5,22 | N 13,18 | Br 12,53% |

3d) *2HBr· H-Gly-Arg-DPA*

5,10 g (8 mmol) der Verbindung 3c wurden gemäss Beispiel 1d mit 32 ml 2N HBr in Eisessig deblockiert. Das nach Aufarbeitung erhaltene Rohprodukt wurde in 100 ml MeOH gelöst und auf einer Säule von Sephadex LH-20 gereinigt. Diejenige Fraktion des MeOH-Eluates, die sich durch Trypsinbehandlung unter Freisetzung von 5-Aminoisophthalsäuredimethylester spalten liess, wurde im Vakuum bei 30° zur Trockne eingeengt. Nach Trocknung des Rückstandes im Vakuumtrockenschrank bei 40° über $P_2O_5$ erhielt man 4,25 g (90,9% der Theorie) der amorphen Verbindung 3d, die im DSC im LMS einheitlich war.

*Elementaranalyse* und Berechnung aus der Bruttoformel $C_{18}H_{28}N_6O_6Br_2$ ergaben die folgenden Werte:

| | | | | |
|---|---|---|---|---|
| gef.: | C 36,85 | H 4,90 | N 14,72 | Br 26,95% |
| ber.: | C 37,00 | H 4,83 | N 14,38 | Br 27,35% |

3e) *Cbo-D-Leu-Gly-Arg-DPA· HBr*

2,92 g (5 mmol) der Verbindung 3d wurden gemäss Beispiel 1e mit 2,13 g (5,5 mmol) Cbo-D-Leu-OpNP umgesetzt. Das nach Aufarbeitung erhaltene Rohprodukt wurde in 100 ml 50%iger AcOH gelöst und auf einer Säule von Sephadex G-15 gereinigt. Diejenige Fraktion des AcOH-Eluates, die sich durch Trypsinbehandlung unter Freisetzung von 5-Aminoisophthalsäuredimethylester spalten liess, wurde im Vakuum bei 40° zur Trockne eingeengt. Nach Trocknung des Rückstandes im Vakuumtrockenschrank bei 60° über $P_2O_5$ erhielt man 3,11 g (82,9% der Theorie) der amorphen Verbindung 3e, die im DSC im LMS einheitlich war.

*Elementaranalyse* und Berechnung aus der Bruttoformel $C_{32}H_{44}N_7O_9Br$ ergaben die folgenden Werte:

| | | | | |
|---|---|---|---|---|
| gef.: | C 50,88 | H 5,99 | N 13,26 | Br 10,48% |
| ber.: | C 51,20 | H 5,91 | N 13,06 | Br 10,64% |

Die Aminosäureanalyse ergab die zu erwartenden Aminosäuren im richtigen Verhältnis:
Gly: 1,00; Leu: 1,00; Arg: 0,97.

*Beispiel 4:*

*Cbo-D-Leu-Sar-Arg-2-NA· HBr*

4b) *2HBr· H-Arg-2-NA*

9,40 g (20 mmol) käufliches Cbo-Arg-2-NA · HCl wurden gemäss Beispiel 1b mit einer Lösung von 80 ml 2N HBr in Eisessig deblockiert. Das nach Aufarbeitung erhaltene Produkt wurde in 150 ml MeOH gelöst und auf einer Säule von Sephadex LH-20 gereinigt. Diejenige Fraktion des MeOH-Eluates, die sich durch Trypsinbehandlung unter Freisetzung von 2-Naphthylamin spalten liess, wurde im Vakuum bei 30° zur Trockne eingeengt. Nach Trocknung des Rückstandes im Vakuumtrockenschrank bei 40° über $P_2O_5$ erhielt man 8,60 g (93,2% der Theorie) der amorphen Verbindung 4b, die im DSC im LMS einheitlich war.

*Elementaranalyse* und Berechnung aus der Bruttoformel $C_{16}H_{23}N_5OBr_2$ ergaben die folgenden Werte:

| | | | | |
|---|---|---|---|---|
| gef.: | C 42,08 | H 5,12 | N 14,68 | Br 33,96% |
| ber.: | C 41,67 | H 5,03 | N 15,19 | Br 34,65% |

4c) *Cbo-Sar-Arg-2-NA· HBr*

4,6 g (10 mmol) der Verbindung 4b wurden gemäss Beispiel 1c mit 3,80 g (11 mmol) Cbo-Sar-OpNP umgesetzt. Das nach Aufarbeitung erhaltene Rohprodukt wurde in 150 ml 50%iger AcOH gelöst und auf einer Säule von Sephadex G-15 gereinigt. Diejenige Fraktion des AcOH-Eluates, die sich durch Trypsinbehandlung unter Freisetzung von 2-Naphthylamin spalten liess, wurde im Vakuum bei 40° zur Trockne eingeengt. Nach Trocknung des Rückstandes im Vakuumtrockenschrank bei 60° über $P_2O_5$ erhielt man 4,95 g (84,5% der Theorie) der amorphen Verbindung 4c, die im DSC im LMS einheitlich war.

*Elementaranalyse* und Berechnung aus der Bruttoformel $C_{27}H_{33}N_6O_4Br$ ergaben die folgenden Werte:

| | | | | |
|---|---|---|---|---|
| gef.: | C 55,72 | H 6,73 | N 14,68 | Br 13,42% |
| ber.: | C 55,39 | H 5,68 | N 14,35 | Br 13,65% |

4d) *2HBr· H-Sar-Arg-2-NA*

4,68 g (8 mmol) der Verbindung 4c wurden gemäss Beispiel 1d mit 28 ml 2N HBr in Eisessig deblockiert. Das nach Aufarbeitung erhaltene Rohprodukt wurde in 100 ml MeOH gelöst und auf einer Säule von Sephadex LH-20 gereinigt. Diejenige Fraktion des MeOH-Eluates, die sich durch Trypsinbehandlung unter Freisetzung von 2-Naphthylamin spalten liess, wurde im Vakuum bei 30° zur Trockne eingeengt. Nach Trocknung des Rückstandes im Vakuumtrockenschrank bei 40° über $P_2O_5$ erhielt man 4,08 g (95,8% der Theorie) der amorphen Verbindung 4d, die im DSC im LMS einheitlich war.

*Elementaranalyse* und Berechnung aus der Bruttoformel $C_{19}H_{28}N_6O_2Br_2$ ergaben die folgenden Werte:

| | | | | |
|---|---|---|---|---|
| gef.: | C 43,9 | H 5,32 | N 16,02 | Br 29,68% |
| ber.: | C 42,87 | H 5,30 | N 15,79 | Br 30,02% |

4e) *Cbo-D-Leu-Sar-Arg-2-NA· HBr*

2,66 g (5 mmol) der Verbindung 4d wurden gemäss Beispiel 1e mit 2,13 g (5,5 mmol) Cbo-D-Leu-OpNP umgesetzt. Das nach Aufarbeitung erhaltene Rohprodukt wurde in 100 ml 50%iger AcOH gelöst und auf einer Säule von Sephadex G-15 gereinigt. Die erste Hauptfraktion des AcOH-Eluates, die sich durch Trypsinbehandlung unter Freisetzung von 2-Naphthylamin spalten liess, wurde im Vakuum bei 40° zur Trockne eingeengt und dann im Vakuumtrockenschrank bei 60° über $P_2O_5$ getrocknet. Man erhielt 3,01 g (86,2% der

Theorie) der amorphen Verbindung 4e, die im DSC im LMS einheitlich war.

*Elementaranalyse* und Berechnung aus der Bruttoformel $C_{33}H_{44}N_7O_6Br$ ergaben die folgenden Werte:

| | | | | |
|---|---|---|---|---|
| gef.: | C 57,05 | H 6,40 | N 14,30 | Br 11,12% |
| ber.: | C 56,73 | H 6,35 | N 14,03 | Br 11,44% |

Die Aminosäureanalyse ergab die zu erwartenden Aminosäuren im richtigen Verhältnis: Sar: 1,00; Leu: 1,02; Arg: 0,97.

*Beispiel 5:*

*Cbo-D-Leu-Sar-Arg-4-MeO-2-NA· HBr*

5b) *2HBr· H-Arg-4-MeO-2-NA*

10,0 g (20 mmol) käufliches Cbo-Arg-4-MeO-2-NA·HCl wurden gemäss Beispiel 1b mit 80 ml 2N HBr in Eisessig deblockiert. Das nach Aufarbeitung erhaltene Rohprodukt wurde in 150 ml MeOH gelöst und auf einer Säule von Sephadex LH-20 gereinigt. Die Hauptfraktion des MeOH-Eluates, die sich durch Trypsinbehandlung unter Freisetzung von 4-Methoxy-2-naphthylamin spalten liess, wurde im Vakuum bei 30° zur Trockne eingeengt. Nach Trocknung des Rückstandes im Vakuumtrockenschrank bei 40° über $P_2O_5$ erhielt man 8,98 g (91,4% der Theorie) der amorphen Verbindung 5b, die im DSC im LMS einheitlich war.

*Elementaranalyse* und Berechnung aus der Bruttoformel $C_{17}H_{25}N_5O_2Br_2$ ergaben die folgenden Werte:

| | | | | |
|---|---|---|---|---|
| ber.: | C 41,22 | H 5,19 | N 14,40 | Br 32,01% |
| gef.: | C 41,57 | H 5,13 | N 14,26 | Br 32,53% |

5c) *Cbo-Sar-Arg-4-MeO-2-NA· HBr*

4,91 g (10 mmol) der Verbindung 5b wurden gemäss Beispiel 1c mit 3,80 g (11 mmol) Cbo-Sar-OpNP umgesetzt. Das nach Aufarbeitung erhaltene Rohprodukt wurde in 150 ml 50%iger AcOH gelöst und auf einer Säule von Sephadex G-15 gereinigt. Die erste Hauptfraktion des AcOH-Eluates, die sich durch Trypsinbehandlung unter Freisetzung von 4-Methoxy-2-naphthylamin spalten liess, wurde im Vakuum bei 40° zur Trockne eingeengt. Nach Trocknung des Rückstandes im Vakuumtrockenschrank bei 60° über $P_2O_5$ erhielt man 4,86 g (79,0% der Theorie) der amorphen Verbindung 5c, die im DSC im LMS einheitlich war.

*Elementaranalyse* und Berechnung aus der Bruttoformel $C_{28}H_{35}N_6O_5Br$ ergaben die folgenden Werte:

| | | | | |
|---|---|---|---|---|
| ber.: | C 54,38 | H 5,81 | N 13,93 | Br 12,75% |
| gef.: | C 54,64 | H 5,73 | N 13,65 | Br 12,98% |

5d) *2HBr· H-Sar-Arg-4-MeO-2-NA*

4,31 g (7 mmol) der Verbindung 5c wurden gemäss Beispiel 1d mit 28 ml 2N HBr in Eisessig deblockiert. Das nach Aufarbeitung erhaltene Rohprodukt wurde in 100 ml MeOH gelöst und auf einer Säule von Sephadex LH-20 gereinigt. Die Hauptfraktion des MeOH-Eluates, die sich durch Trypsinbehandlung unter Bildung von 4-Methoxy-2-naphthylamin spalten liess, wurde im Vakuum bei 30° zur Trockne eingeengt. Nach Trocknung des Rückstandes im Vakuumtrockenschrank bei 40° über $P_2O_5$ erhielt man 3,74 g (95,0% der Theorie) der amorphen Verbindung 5d, die im DSC im LMS einheitlich war.

*Elementaranalyse* und Berechnung aus der Bruttoformel $C_{20}H_{30}N_6O_3Br_2$ ergaben die folgenden Werte:

| | | | | |
|---|---|---|---|---|
| gef.: | C 43,01 | H 5,44 | N 15,25 | Br 28,03% |
| ber.: | C 42,72 | H 5,38 | N 14,95 | Br 28,42% |

Die Aminosäureanalyse ergab die zu erwartenden Aminosäuren im richtigen Verhältnis: Sar: 1,00; Leu: 1,01; Arg: 0,98.

5e) *Cbo-D-Leu-Sar-Arg-4-MeO-2-NA· HBr*

2,81 g (5 mmol) der Verbindung 5d wurden gemäss Beispiel 1e mit 2,13 g (5,5 mmol) Cbo-D-Leu-OpNP umgesetzt. Das nach Aufarbeitung erhaltene Rohprodukt wurde in 125 ml 50%iger AcOH gelöst und auf einer Säule von Sephadex G-15 gereinigt. Die erste Hauptfraktion des AcOH-Eluates, die sich durch Trypsinbehandlung unter Freisetzung von 4-Methoxy-2-naphthylamin spalten liess, wurde im Vakuum bei 40° zur Trockne eingeengt. Nach Trocknung des Rückstandes im Vakuumtrockenschrank bei 60° über $P_2O_5$ erhielt man 2,98 g (81,8% der Theorie) der amorphen Verbindung 5e, die im DSC im LMS einheitlich war.

*Elementaranalyse* und Berechnung aus der Bruttoformel $C_{34}H_{46}N_7O_6Br$ ergaben die folgenden Werte:

| | | | | |
|---|---|---|---|---|
| gef.: | C 56,28 | H 6,34 | N 13,75 | Br 10,68% |
| ber.: | C 56,04 | H 6,36 | N 13,46 | Br 10,97% |

*Beispiel 6:*

*Cbo-D-Leu-Gly-Arg-pNA· AcOH*

6,80 g (10 mmol) Cbo-D-Leu-Gly-Arg-pNA·HBr (hergestellt gemäss Beispiel 1) wurden in 75 ml 60%igem wässerigem MeOH gelöst. Die Lösung wurde auf eine Säule von Amberlite JRA-401 in der Acetatform gegeben. Die Säule wurde mittels 60%igem wässerigem MeOH eluiert, wobei durch Ionenaustausch HBr durch AcOH ersetzt wurde. Das Eluat wurde im Vakuum bei 40° zur Trockne eingeengt. Nach dem Trocknen im Vakuumtrockenschrank bei 40° über $P_2O_5$ erhielt man 6,62 g bromidfreies Cbo-D-Leu-Gly-Arg-pNA·AcOH (99,0% der Theorie).

Nach dieser Methode kann man aus dem oben genannten Tripeptidderivat entsprechend andere Salze mit organischen Säuren, z.B. Ameisen-, Propion-, Oxal-, Wein-, Zitronen-, Milch-, Benzoe-, Chlorbenzoe-, Salicyl- oder Phthalsäure, herstellen. Man kann als Ionenaustauscher z.B. Amberlite JRA-401 in der Hydrochloridform verwenden und in die gewünschte Säuresalzform überführen, indem man den genannten Ionenaustauscher durch Behandlung mit Natronlauge in die basische OH-Form überführt und dann mit einer Lösung eines 1:1-Gemisches der gewünschten organischen Säure und deren Natriumsalz in 60%igem wässerigem MeOH behandelt.

*Beispiel 7:*

*Tos-D-Leu-Gly-Arg-pNA·HBr*

6,26 g (10 mmol) 2HBr·H-D-Leu-Gly-Arg-pNA (hergestellt gemäss Beispiel 7f, s. Tabelle 3) wurden in 30 ml destilliertem DMF gelöst. Nach Kühlung auf −15° wurden der Lösung unter Rühren und Feuchtigkeitsausschluss zuerst 2,80 ml (20 mmol) $Et_3N$ und gleich anschliessend 1,91 g (10 mmol) Tosylchlorid zugesetzt. Nach einer Reaktionszeit von etwa 2 bis 3 h bei −15° liess man das Reaktionsgemisch über Nacht bei Raumtemperatur weiterreagieren. Das Reaktionsgemisch wurde wieder auf −15° gekühlt. Das ausgeschiedene Gemisch von $Et_3N \cdot HBr$ und $Et_3N \cdot HCl$ wurde abfiltriert und mit einer kleinen Menge gekühltem DMF nachgewaschen. Filtrat und Waschlösung wurden vereinigt und im Vakuum bei 50° zur Trockne eingeengt. Der Rückstand wurde in 75 ml 50%iger AcOH gelöst. Die Lösung wurde auf einer mit 50%iger AcOH äquilibrierten Säule von Sephadex G-15 gereinigt. Diejenige Hauptfraktion des AcOH-Eluates, die sich durch Trypsinbehandlung unter Freisetzung von p-Nitroanilin spalten liess, wurde im Vakuum bei 40° zur Trockne eingeengt. Der Rückstand wurde in 100 ml MeOH gelöst, worauf die Lösung nochmals zur Trockne eingeengt wurde. Nach Trocknung des Rückstandes im Vakuumtrockenschrank bei 60° über $P_2O_5$ erhielt man 6,65 g (95,0% der Theorie) der amorphen Verbindung Tos-D-Leu-Gly-Arg-pNA·HBr, die im DSC im LMS einheitlich war.

*Elementaranalyse* und Berechnung aus der Bruttoformel $C_{27}H_{39}N_8SO_7Br$ ergaben die folgenden Werte:

gef.: C 46,85 H 5,66 N 16,28%
ber.: C 46,35 H 5,62 N 16,02%
gef.: S 4,43 Br 11,22%
ber.: S 4,58 Br 11,42%

Anstelle des Tosylchlorids als Acylierungsmittel kann man auch entsprechende Mengen Acetylchlorid, Butyrylchlorid, Octanoylchlorid, Benzoesäurechlorid, p-Methylbenzoesäurechlorid, 2-Chlorbenzoesäurechlorid, Methansulfonylchlorid, n-Butansulfonylchlorid, Phenylsulfonylchlorid, α-Naphthylsulfonylchlorid, Chlorameisensäuremethylester, Chlorameisensäureisobutylester, Chlorameisensäureoctylester, Benzoesäureanhydrid, Essigsäureanhydrid, Phenylessigsäure-OpNP, Phenylpropionsäure-OpNP, Cyclohexylcarbonsäure-OpNP, N-Cbo-4-Aminomethylcyclohexylcarbonsäure-OpNP, N-Cbo-ω-Amino-n-buttersäure-OpNP, N-Cbo-ω-Aminooctansäure-OpNP oder N-Cbo-4-Aminomethylbenzoesäure-OpNP verwenden.

Tabelle 1

| Bsp. | Endprodukt | Ausgangsprodukte (mmol) | Methode Ausbeute (%) | Elementaranalyse gef. (%) | ber. (%) | Aminosäureanalyse | | |
|---|---|---|---|---|---|---|---|---|
| 8 | Cbo-D-Nleu-Gly-Arg-pNA·HBr $C_{28}H_{39}N_8O_7Br$ | 1d (5 mmol) Cbo-D-Nleu-OpNP (5,5 mmol) | Bsp. 1e 82,8 | C 48,95<br>H 5,83<br>N 16,80<br>Br 11,55 | 49,49<br>5,78<br>16,49<br>11,76 | Gly 1,00 | Nleu 0,99 | Arg 0,97 |
| 9 | Cbo-D-Nval-Gly-Arg-pNA·HBr $C_{27}H_{37}N_8O_7Br$ | 1d (5 mmol) Cbo-D-Nval-OpNP (5,5 mmol) | Bsp. 1e 84,2 | C 48,58<br>H 5,66<br>N 16,98<br>Br 11,86 | 48,73<br>5,60<br>16,84<br>12,01 | Gly 1,00 | Nval 1,02 | Arg 0,98 |
| 10 | Cbo-D-But-Gly-Arg-pNA·HBr $C_{26}H_{35}N_8O_7Br$ | 1d (5 mmol) Cbo-D-But-OpNP (5,5 mmol) | Bsp. 1e 83,8 | C 47,73<br>H 5,48<br>N 17,48<br>Br 12,11 | 47,93<br>5,41<br>17,20<br>12,26 | Gly 1,00 | But 0,99 | Arg 0,97 |
| 11 | Cbo-D-Ala-Gly-Arg-pNA·HBr $C_{25}H_{33}N_8O_7Br$ | 1d (5 mmol) Cbo-D-Ala-OpNP (5,5 mmol) | Bsp. 1e 80,5 | C 46,88<br>H 5,26<br>N 17,85<br>Br 12,33 | 47,10<br>5,22<br>17,58<br>12,53 | Gly 1,00 | Ala 1,01 | Arg 0,98 |
| 12 | Cbo-D-CHA-Gly-Arg-pNA·HBr $C_{30}H_{41}N_8O_7Br$ | 1d (5 mmol) Cbo-D-CHA-OpNP (5,5 mmol) | Bsp. 1e 82,7 | C 50,75<br>H 5,93<br>N 16,10<br>Br 11,18 | 51,07<br>5,86<br>15,88<br>11,32 | Gly 1,00 | CHA 0,98 | Arg 0,99 |

Tabelle 1 *(Fortsetzung)*

| Bsp. | Endprodukt | Ausgangs-produkte (mmol) | Methode Aus-beute (%) | Elementaranalyse gef. (%) | ber. (%) | Aminosäureanalyse | | |
|---|---|---|---|---|---|---|---|---|
| 13 | Cbo-D-CHT-Gly-Arg-pNA · HBr<br>$C_{30}H_{41}N_8O_8Br$ | 1d (5 mmol)<br>Cbo-D-CHT-OpNP<br>(5,5 mmol) | Bsp. 1e<br>82,0 | C 49,62<br>H 5,80<br>N 15,76<br>Br 10,88 | 49,93<br>5,73<br>15,53<br>11,07 | Gly<br>1,00 | CHT<br>0,96 | Arg<br>1,01 |
| 14 | Cbo-D-Ph'Gly-Gly-Arg-pNA · HBr<br>$C_{29}H_{33}N_8O_7Br$ | 1d (5 mmol)<br>Cbo-D-Ph'Gly-OpNP<br>(5,5 mmol) | Bsp. 1e<br>81,3 | C 50,60<br>H 4,88<br>N 16,63<br>Br 11,48 | 50,81<br>4,85<br>16,35<br>11,66 | Gly<br>1,00 | Ph'Gly<br>0,98 | Arg<br>0,98 |
| 15 | Cbo-D-Phe-Gly-Arg-pNA · HBr<br>$C_{30}H_{35}N_8O_7Br$ | 1d (5 mmol)<br>Cbo-D-Phe-OpNP<br>(5,5 mmol) | Bsp. 1e<br>80,0 | C 51,20<br>H 5,07<br>N 16,28<br>Br 11,35 | 51,51<br>5,04<br>16,02<br>11,42 | Gly<br>1,00 | Phe<br>1,01 | Arg<br>0,98 |
| 16 | Cbo-D-Ile-Gly-Arg-pNA · HBr<br>$C_{28}H_{39}N_8O_7Br$ | 1d (5 mmol)<br>Cbo-D-Ile-OpNP<br>(5,5 mmol) | Bsp. 1e<br>81,2 | C 49,12<br>H 5,80<br>N 16,66<br>Br 11,58 | 49,49<br>5,78<br>16,49<br>11,76 | Gly<br>1,00 | Ile<br>0,99 | Arg<br>0,98 |
| 17 | Cbo-D-Glu (γ-OtBu)-Gly-Arg-pNA · HBr<br>$C_{31}H_{43}N_8O_9Br$ | 1d (5 mmol)<br>Cbo-D-Glu(γ-OtBu)-OpNP<br>(5,5 mmol) | Bsp. 1e<br>78,2 | C 49,18<br>H 5,82<br>N 15,12<br>Br 10,50 | 49,54<br>5,77<br>14,91<br>10,63 | Gly<br>1,00 | Glu<br>0,96 | Arg<br>0,98 |
| 18 | Cbo-D-Asp(β-OtBu)-Gly-Arg-pNA · HBr<br>$C_{30}H_{41}N_8O_9Br$ | 1d (5 mmol)<br>Cbo-D-Asp(β-OtBu)-OpNP<br>(5,5 mmol) | Bsp. 1e<br>77,6 | C 48,70<br>H 5,66<br>N 15,02<br>Br 10,65 | 48,85<br>5,60<br>15,19<br>10,83 | Gly<br>1,00 | Asp<br>0,97 | Arg<br>1,00 |
| 19 | Cbo-D-His-Gly-Arg-pNA · 2HBr<br>$C_{28}H_{36}N_{10}O_7Br_2$ | 1d (5 mmol)<br>Cbo-D-His-OpNP<br>(5,5 mmol) | Bsp. 1e<br>68,5 | C 42,71<br>H 4,70<br>N 18,05<br>Br 20,12 | 42,87<br>4,63<br>17,86<br>20,37 | Gly<br>1,00 | His<br>0,94 | Arg<br>0,99 |
| 20 | Cbo-D-Leu-Sar-Arg-pNA · HBr<br>$C_{29}H_{41}N_8O_7Br$ | 20d (5 mmol)<br>Cbo-D-Leu-OpNP<br>(5,5 mmol) | Bsp. 1e<br>82,2 | C 50,48<br>H 6,02<br>N 16,38<br>Br 11,33 | 50,22<br>5,96<br>16,16<br>11,52 | Sar<br>1,00 | Leu<br>1,01 | Arg<br>0,97 |
| 21 | Cbo-D-Leu-Ala-Arg-pNA · HBr<br>$C_{29}H_{41}N_8O_7Br$ | 21d (5 mmol)<br>Cbo-D-Leu-OpNP<br>(5,5 mmol) | Bsp. 1e<br>83,5 | C 50,48<br>H 6,03<br>N 16,28<br>Br 11,40 | 50,22<br>5,96<br>16,16<br>11,52 | Ala<br>1,00 | Leu<br>1,01 | Arg<br>0,98 |
| 22 | $CH_3SO_2$-D-Nleu-Gly-Arg-pNA · AcOH<br>$C_{23}H_{38}N_8O_9S$ | 34f (3 mmol)<br>$CH_3SO_2$-Cl<br>(3,3 mmol) | Bsp. 7 und 6<br>83,1 | C 45,66<br>H 6,41<br>N 18,93<br>S 5,24 | 45,84<br>6,36<br>18,59<br>5,32 | Gly<br>1,00 | Nleu<br>1,01 | Arg<br>0,98 |
| 23 | Isobutoxy-CO-D-Nleu-Gly-Arg-pNA · AcOH<br>$C_{27}H_{44}N_8O_9$ | 34f (3 mmol)<br>Isobutoxy-CO-Cl<br>(3,3 mmol) | Bsp. 7 und 6<br>81,4 | C 51,63<br>H 7,12<br>N 18,17 | 51,91<br>7,10<br>17,94 | Gly<br>1,00 | Nleu<br>1,02 | Arg<br>0,99 |

Tabelle 1 *(Fortsetzung)*

| Bsp. | Endprodukt | Ausgangs-produkte (mmol) | Methode Aus-beute (%) | Elementaranalyse gef. (%) | ber. (%) | Aminosäureanalyse | | |
|---|---|---|---|---|---|---|---|---|
| 24 | Cbo-D-Leu-N(Et)-Gly-Arg-pNA · HBr $C_{30}H_{43}N_8O_7Br$ | 24d (5 mmol) Cbo-D-Leu-OpNP (5,5 mmol) | Bsp. 1e 80,6 | C 51,30<br>H 6,15<br>N 16,10<br>Br 11,12 | 50,92<br>6,13<br>15,84<br>11,29 | Leu 1,00 | N(Et)Gly 0,98 | Arg 0,99 |
| 25 | Cbo-D-Leu-N(Pr)Gly-Arg-pNA · HBr $C_{31}H_{45}N_8O_7Br$ | 25d (5 mmol) Cbo-D-Leu-OpNP (5,5 mmol) | Bsp. 1e 77,5 | C 51,95<br>H 6,34<br>N 15,80<br>Br 10,83 | 51,59<br>6,29<br>15,53<br>11,07 | Leu 1,00 | N(Pr)Gly 0,96 | Arg 0,98 |
| 26 | Cbo-D-CHA-Sar-Arg-pNA · HBr $C_{31}H_{43}N_8O_7Br$ | 20d (5 mmol) Cbo-D-CHA-OpNP (5,5 mmol) | Bsp. 1e 84,2 | C 51,48<br>H 6,08<br>N 15,76<br>Br 10,95 | 51,74<br>6,02<br>15,57<br>11,10 | Sar 1,00 | CHA 0,98 | Arg 0,98 |
| 27 | Cbo-D-CHT-Sar-Arg-pNA · HBr $C_{31}H_{43}N_8O_8Br$ | 20d (5 mmol) Cbo-D-CHT-OpNP (5,5 mmol) | Bsp. 1e 80,9 | C 50,33<br>H 5,96<br>N 15,48<br>Br 10,70 | 50,61<br>5,89<br>15,23<br>10,86 | Sar 1,00 | CHT 0,97 | Arg 0,99 |
| 28 | Cbo-D-Nleu-Sar-Arg-pNA · HBr $C_{29}H_{41}N_8O_7Br$ | 20d (5 mmol) Cbo-D-Nleu-OpNP (5,5 mmol) | Bsp. 1e 80,6 | C 50,31<br>H 6,00<br>N 16,40<br>Br 11,38 | 50,22<br>5,96<br>16,16<br>11,52 | Sar 1,00 | Nleu 1,02 | Arg 0,98 |
| 29 | Cbo-D-Nval-Sar-Arg-pNA · HBr $C_{28}H_{39}N_8O_7Br$ | 20d (5 mmol) Cbo-D-Nval-OpNP | Bsp. 1e 82,7 | C 49,33<br>H 5,80<br>N 16,74<br>Br 11,62 | 49,49<br>5,78<br>16,49<br>11,76 | Sar 1,00 | Nval 1,00 | Arg 0,98 |
| 30 | Benzolsulfonyl-D-Leu-Gly-Arg-pNA · HBr $C_{26}H_{37}N_8O_7SBr$ | 7f (5 mmol) Benzolsulfochlorid (5 mmol) | Bsp. 7g 92 | C 45,85<br>H 5,47<br>N 16,58<br>S 4,53<br>Br 11,30 | 45,55<br>5,44<br>16,34<br>4,68<br>11,65 | Gly 1,00 | Leu 1,01 | Arg 0,99 |
| 31 | Methansulfonyl-D-Leu-Gly-Arg-pNA · HBr $C_{21}H_{35}N_8O_7SBr$ | 7f (5 mmol) Methansulfochlorid (5 mmol) | Bsp. 7g 90,5 | C 40,90<br>H 5,71<br>N 18,28<br>S 5,01<br>Br 12,55 | 40,45<br>5,66<br>17,97<br>5,14<br>12,82 | Gly 1,00 | Leu 1,02 | Arg 0,99 |
| 32 | α-Naphtalinsulfonyl-D-Leu-Gly-Arg-pNA · HBr $C_{30}H_{39}N_8O_7SBr$ | 7f (5 mmol) α-Naphtalinsulfochlorid (5 mmol) | Bsp. 7g 88 | C 49,38<br>H 5,40<br>N 15,55<br>S 4,25<br>Br 10,62 | 48,98<br>5,34<br>15,23<br>4,36<br>10,86 | Gly 1,00 | Leu 1,01 | Arg 0,98 |
| 33 | n-Butansulfonyl-D-Leu-Gly-Arg-pNA · HBr $C_{24}H_{41}N_8O_7SBr$ | 7f (5 mmol) n-Butansulfochlorid (5 mmol) | Bsp. 7g 88,3 | C 43,55<br>H 6,28<br>N 17,08<br>S 4,73<br>Br 11,84 | 43,31<br>6,21<br>16,84<br>4,82<br>12,00 | Gly 1,00 | Leu 0,99 | Arg 0,99 |

Tabelle 1 *(Fortsetzung)*

| Bsp. | Endprodukt | Ausgangs-produkte (mmol) | Methode Ausbeute (%) | Elementaranalyse gef. (%) | ber. (%) | Aminosäureanalyse | | |
|---|---|---|---|---|---|---|---|---|
| 34 | Ac-D-Nleu-Gly-Arg-pNA · HBr $C_{22}H_{35}N_8O_6Br$ | 34f (5 mmol) Essigsäureanhydrid (6 mmol) | Bsp. 7g 78,5 | C 45,18 H 6,08 N 19,38 Br 13,35 | 44,98 6,01 19,07 13,60 | Gly 1,00 | Nleu 1,01 | Arg 1,00 |
| 35 | n-Butyryl-D-Nleu-Gly-Arg-pNA · HBr $C_{24}H_{39}N_8O_6Br$ | 34f (5 mmol) n-Butyrylchlorid (5 mmol) | Bsp. 7g 81,2 | C 47,03 H 6,45 N 18,41 Br 15,38 | 46,83 6,39 18,21 15,60 | Gly 1,00 | Nleu 0,97 | Arg 0,99 |
| 36 | n-Octanoyl-D-Nleu-Gly-Arg-pNA · HBr $C_{28}H_{47}N_8O_6Br$ | 34f (5 mmol) n-Octanoylchlorid (5 mmol) | Bsp. 7g 78,1 | C 49,88 H 7,11 N 16,79 Br 11,65 | 50,07 7,01 16,68 11,90 | Gly 1,00 | Leu 1,01 | Arg 0,98 |
| 37 | 2-Phenylacetyl-D-Leu-Sar-Arg-pNA · HBr $C_{29}H_{41}N_8O_6Br$ | 37f (5 mmol) 2-Phenylessigsäure-OpNP (5,5 mmol) | Bsp. 7g 80,4 | C 51,62 H 6,12 N 16,80 Br 11,53 | 51,40 6,10 16,54 11,79 | Sar 1,00 | Leu 1,02 | Arg 1,00 |
| 38 | 4-Phenylbutyryl-D-Leu-Sar-Arg-pNA · HBr $C_{31}H_{45}N_8O_6Br$ | 37f (5 mmol) 4-Phenylbuttersäure-OpNP (5,5 mmol) | Bsp. 7g 79,2 | C 52,99 H 6,47 N 16,05 Br 11,18 | 52,76 6,43 15,88 11,32 | Sar 1,00 | Leu 0,99 | Arg 0,98 |
| 39 | Cyclohexylcarbonyl-D-Leu-Sar-Arg-pNA · HBr $C_{28}H_{45}N_8O_6Br$ | 37f (5 mmol) Cyclohexancarbonsäure-OpNP (5,5 mmol) | Bsp. 7g 82,3 | C 50,51 H 6,81 N 16,75 Br 11,81 | 50,22 6,77 16,73 11,93 | Sar 1,00 | Leu 1,01 | Arg 1,00 |
| 40 | Cbo-4-aminomethylcyclohexylcarbonyl-D-Leu-Sar-Arg-pNA · HBr $C_{37}H_{54}N_9O_8Br$ | 37f (5 mmol) Cbo-4-aminomethylhexancarbonsäure-OpNP (5 mmol) | Bsp. 7g 84,1 | C 53,68 H 5,56 N 15,30 Br 9,31 | 53,36 6,54 15,14 9,59 | Sar 1,00 | Leu 0,99 | Arg 0,98 |
| 41 | 4-Aminomethylcyclohexylcarbonyl-D-Leu-Sar-Arg-pNA · 2HBr $C_{29}H_{49}N_9O_6Br_2$ | 40 (3 mmol) 2H HBr/AcOH | Bsp. 1d 96,2 | C 44,51 H 6,39 N 16,38 Br 20,18 | 44,68 6,34 16,17 20,50 | Sar 1,00 | Leu 0,99 | Arg 0,98 |
| 42 | Cbo-Gly-D-Nleu-Sar-Arg-pNA · HBr $C_{31}H_{44}N_9O_8Br$ | 42f (5 mmol) Cbo-Gly-OpNP (5,5 mmol) | Bsp. 1c 77,8 | C 49,88 H 5,96 N 16,93 Br 10,44 | 49,60 5,91 16,79 10,64 | Sar 1,00 | Nleu 0,98 | Arg 0,99 |
| 43 | H-Gly-D-Nleu-Sar-Arg-pNA · 2HBr $C_{23}H_{39}N_9O_6Br_2$ | 42 (3 mmol) 2N HBr/AcOH | Bsp. 1d 93,6 | C 39,88 H 5,69 N 18,18 Br 22,52 | 39,61 5,64 18,08 22,91 | Sar 1,00 | Nleu 1,00 | Arg 0,98 |
| 44 | Cbo-ω-Aminobutyryl-D-Nleu-Sar-Arg-pNA · HBr $C_{33}H_{48}N_9O_8Br$ | 42f (5 mmol) Cbo-4-aminobutyryl-OpNP (5,5 mmol) | Bsp. 7g 77,2 | C 50,49 H 6,26 N 16,39 Br 10,11 | 50,90 6,21 16,19 10,26 | Sar 1,00 | Nleu 0,99 | Arg 0,98 |

Tabelle 1 (Fortsetzung)

| Bsp. | Endprodukt | Ausgangs-produkte (mmol) | Methode Aus-beute (%) | Elementaranalyse gef. (%) | ber. (%) | Aminosäureanalyse | | |
|---|---|---|---|---|---|---|---|---|
| 45 | ω-Aminobutyryl-D-Nleu-Sar-Arg-pNA · 2HBr $C_{25}H_{43}N_9O_6Br_2$ | 44 (3 mmol) 2N HBr/AcOH | Bsp. 1d 90,8 | C 41,65<br>H 6,03<br>N 17,39<br>Br 21,75 | 41,39<br>5,97<br>17,38<br>22,03 | Sar 1,00 | Nleu 1,01 | Arg 0,98 |
| 46 | Cbo-ω-Amino-octanoyl-D-Nleu-Sar-Arg-pNA · HBr $C_{37}H_{56}N_9O_8Br$ | 42f (5 mmol) Cbo-8-amino-octanoyl-OpNP (5,5 mmol) | Bsp. 7g 76,2 | C 53,09<br>H 6,80<br>N 14,88<br>Br 9,45 | 53,23<br>6,76<br>15,10<br>9,57 | Sar 1,00 | Nleu 0,98 | Arg 0,99 |
| 47 | ω-Aminooctanoyl-D-Nleu-Sar-Arg-pNA · 2HBr $C_{29}H_{51}N_9O_6Br$ | 46 (3 mmol) 2N Hbr/AcOH | Bsp. 1d 92,8 | C 44,95<br>H 6,59<br>N 16,31<br>Br 20,12 | 44,56<br>6,58<br>16,13<br>20,45 | Sar 1,00 | Nleu 0,98 | Arg 1,00 |
| 48 | Bz-D-Nval-Gly-Arg-pNA · HBr $C_{26}H_{35}N_8O_6Br$ | 48f (5 mmol) Benzoesäurean-hydrid ($Bz_2O$) (7,5 mmol) | Bsp. 7g 77,5 | C 49,25<br>H 5,59<br>N 17,90<br>Br 12,33 | 49,14<br>5,55<br>17,63<br>12,57 | Gly 1,00 | Nval 0,99 | Arg 0,98 |
| 49 | 4-Methylbenzoyl-D-Nval-Gly-Arg-pNA · HBr $C_{27}H_{37}N_8O_6Br$ | 48f (5 mmol) 4-Methylbenzoe-säureanhydrid (7,5 mmol) | Bsp. 7g 76,8 | C 50,18<br>H 5,78<br>N 17,35<br>Br 12,18 | 49,93<br>5,74<br>17,25<br>12,30 | Gly 1,00 | Nval 1,01 | Arg 0,98 |
| 50 | 2-Chlorbenzoyl-D-Nval-Gly-Arg-pNA · HCl $C_{26}H_{34}N_8O_6Cl_2$ | 48f (5 mmol) 2-Chlorbenzoe-säureanhydrid (7,5 mmol) | Bsp. 7g 76,4 | C 50,33<br>H 5,46<br>N 18,09<br>Cl 11,18 | 49,92<br>5,48<br>17,91<br>11,34 | Gly 1,00 | Nval 0,99 | Arg 0,99 |
| 51 | 2-(4-Cbo-amino)-phenylacetyl-D-Nval-Gly-Arg-pNA · HBr $C_{35}H_{44}N_9O_8Br$ | 48f (5 mmol) 2-(4-Cbo-ami-no)-Phenylacetyl-OpNP (5,5 mmol) | Bsp. 7g 77,9 | C 52,48<br>H 5,52<br>N 15,94<br>Br 9,78 | 52,63<br>5,55<br>15,78<br>10,00 | Gly 1,00 | Nval 0,98 | Arg 0,98 |
| 52 | 2-(4-Amino)-phenylacetyl-D-Nval-Gly-Arg-pNA · 2HBr $C_{35}H_{44}N_9O_8Br_2$ | 55 (3 mmol) 2N HBr/AcOH | Bsp. 1d 91,8 | C 43,17<br>H 5,24<br>N 17,09<br>Br 21,18 | 43,50<br>5,27<br>16,91<br>21,44 | Gly 1,00 | Nval 0,99 | Arg 1,00 |
| 53 | $CH_3O$-CO-D-Nval-Sar-Arg-pNA · HBr $C_{22}H_{35}N_8O_7Br$ | 53f (5 mmol) Chlorameisen-säuremethylester (5,5 mmol) | Bsp. 7g 83,6 | C 43,59<br>H 5,92<br>N 18,76<br>Br 12,95 | 43,79<br>5,85<br>18,57<br>13,24 | Sar 1,00 | Nval 0,98 | Arg 0,99 |
| 54 | $CH_3$-$(CH_2)_7$-O-CO-D-Nval-Sar-Arg-pNA · HBr $C_{29}H_{49}N_8O_7Br$ | 53f (5 mmol) Chlorameisen-säureoctylester (5,5 mmol) | Bsp. 7g 79,8 | C 49,51<br>H 7,08<br>N 16,22<br>Br 11,19 | 49,64<br>7,04<br>15,97<br>11,39 | Sar 1,00 | Nval 1,00 | Arg 0,98 |
| 55 | BOC-D-Leu-Gly-Arg-pNA · HBr $C_{25}H_{41}N_8O_7Br$ | 1d (5 mmol) BOC-D-Leu-OpNP (5,5 mmol) | Bsp. 1e 84,2 | C 46,88<br>H 6,41<br>N 17,71<br>Br 12,20 | 46,51<br>6,40<br>17,36<br>12,38 | Gly 1,00 | Leu 1,00 | Arg 0,99 |

Tabelle 1 *(Fortsetzung)*

| Bsp. | Endprodukt | Ausgangs-produkte (mmol) | Methode Aus-beute (%) | Elementaranalyse gef. (%) | Elementaranalyse ber. (%) | Aminosäureanalyse | | |
|---|---|---|---|---|---|---|---|---|
| 56 | 4-MeO-Cbo-D-Leu-Gly-Arg-pNA · HBr<br>$C_{29}H_{41}N_8O_8Br$ | 1d (5 mmol)<br>4-MeO-Cbo-D-Leu-OpNP<br>(5,5 mmol) | Bsp. 1e<br>80,5 | C 48,81<br>H 5,90<br>N 16,00<br>Br 11,05 | 49.09<br>5,82<br>15,79<br>11,26 | Gly<br>1,00 | Leu<br>1,01 | Arg<br>0,98 |
| 57 | $CH_3O$-CO-D-CHA-Gly-Arg-pNA · AcOH<br>$C_{27}H_{42}N_8O_9$ | 57f (5 mmol)<br>$CH_3$-CO-Cl<br>(5,5 mmol) | Bsp. 7<br>und 6<br>85,6 | C 52,60<br>H 6,88<br>N 18,30 | 52,08<br>6,80<br>18,00 | Gly<br>1,00 | CHA<br>0,97 | Arg<br>0,99 |
| 58 | $C_2H_5O$-CO-D-CHA-Gly-Arg-pNA · AcOH<br>$C_{28}H_{44}N_8O_9$ | 57f (5 mmol)<br>$C_2H_5O$-CO-Cl<br>(5,5 mmol) | Bsp. 7<br>und 6<br>84,3 | C 52,48<br>H 7,03<br>N 17,75 | 52,82<br>6,97<br>17,60 | Gly<br>1,00 | CHA<br>0,98 | Arg<br>0,98 |
| 59 | $CH_3SO_2$-D-CHA-Gly-Arg-pNA · AcOH<br>$C_{26}H_{42}N_8O_9S$ | 57f (5 mmol)<br>$CH_3SO_2$-Cl<br>(5,5 mmol) | Bsp. 7<br>und 6<br>86,8 | C 48,22<br>H 6,66<br>N 17,58<br>S 4,80 | 48,59<br>6,59<br>17,44<br>4,99 | Gly<br>1,00 | CHA<br>0,97 | Arg<br>0,97 |
| 60 | Cbo-D-Leu-Gly-Arg-2-NA · HBr<br>$C_{32}H_{42}N_7O_6Br$ | 60d (5 mmol)<br>Cbo-D-Leu-OpNP<br>(5,5 mmol) | Bsp. 1e<br>79,5 | C 55,78<br>H 6,24<br>N 14,48<br>Br 11,45 | 56,14<br>6,18<br>14,32<br>11,67 | Gly<br>1,00 | Leu<br>1,01 | Arg<br>0,99 |
| 61 | $CH_3O$-CO-D-Leu-Gly-Arg-2-NA · AcOH<br>$C_{28}H_{41}N_7O_7$ | 60f (5 mmol)<br>$CH_3O$-CO-Cl<br>(5,5 mmol) | Bsp. 7<br>und 6<br>75,9 | C 57,02<br>H 7,10<br>N 16,83 | 57,23<br>7,03<br>16,68 | Gly<br>1,00 | Leu<br>1,00 | Arg<br>0,98 |
| 62 | $CH_3SO_2$-D-Leu-Gly-Arg-2-NA · AcOH<br>$C_{27}H_{41}N_7O_7S$ | 60f (5 mmol)<br>$CH_3SO_2$-Cl<br>(5 mmol) | Bsp. 7<br>und 6<br>82,1 | C 53,08<br>H 6,84<br>N 16,25<br>S 5,15 | 53,36<br>6,80<br>16,13<br>5,28 | Gly<br>1,00 | Leu<br>1,01 | Arg<br>0,98 |
| 63 | Cbo-D-Leu-Gly-Arg-4-MeO-2-NA · HBr<br>$C_{33}H_{44}N_7O_6Br$ | 63d (5 mmol)<br>Cbo-D-Leu-OpNP<br>(5,5 mmol) | Bsp. 1e<br>84,0 | C 55,16<br>H 6,25<br>N 13,96<br>Br 11,00 | 55,46<br>6,21<br>13,72<br>11,18 | Gly<br>1,00 | Leu<br>1,01 | Arg<br>0,99 |
| 64 | $CH_3O$-CO-D-Leu-Gly-Arg-4-MeO-2-NA · AcOH<br>$C_{29}H_{43}N_7O_8$ | 63f (5 mmol)<br>$CH_3O$-CO-Cl<br>(5,5 mmol) | Bsp. 7<br>und 6<br>82,4 | C 56,18<br>H 7,08<br>N 16,09 | 56,39<br>7,02<br>15,87 | Gly<br>1,00 | Leu<br>0,99 | Arg<br>0,99 |
| 65 | $CH_3SO_2$-D-Leu-Gly-Arg-4-MeO-2-NA · AcOH<br>$C_{28}H_{43}N_7O_8S$ | 63f (5 mmol)<br>$CH_3SO_2$-Cl<br>(5,5 mmol) | Bsp. 7<br>und 6<br>77,4 | C 52,58<br>H 6,85<br>N 15,58<br>S 4,92 | 52,73<br>6,80<br>15,37<br>5,03 | Gly<br>1,00 | Leu<br>1,01 | Arg<br>0,98 |
| 66 | $CH_3O$-CO-D-Leu-Gly-Arg-DPA · AcOH<br>$C_{28}H_{43}N_7O_{11}$ | 66f (5 mmol)<br>$CH_3O$-CO-Cl<br>(5,5 mmol) | Bsp. 7<br>und 6<br>79,3 | C 51,23<br>H 6,65<br>N 15,18 | 51,45<br>6,63<br>15,00 | Gly<br>1,00 | Leu<br>0,99 | Arg<br>0,99 |

Tabelle 1 *(Fortsetzung)*

| Bsp. | Endprodukt | Ausgangsprodukte (mmol) | Methode Ausbeute (%) | Elementaranalyse gef. (%) | Elementaranalyse ber. (%) | Aminosäureanalyse | | |
|---|---|---|---|---|---|---|---|---|
| 67 | $CH_3SO_2$-D-Leu-Gly-Arg-DPA · AcOH<br>$C_{27}H_{43}N_7O_{11}S$ | 66f (5 mmol)<br>$CH_3SO_2$-Cl<br>(5,5 mmol) | Bsp. 7 und 6<br>81,2 | C 47,88<br>H 6,50<br>N 14,75<br>S 4,68 | 48,13<br>6,43<br>14,55<br>4,76 | Gly<br>1,00 | Leu<br>1,00 | Arg<br>0,98 |
| 68 | $CH_3O$-CO-D-Leu-Gly-Arg-MCA · AcOH<br>$C_{28}H_{41}N_7O_9$ | 68f (5 mmol)<br>$CH_3O$-CO-Cl<br>(5,5 mmol) | Bsp. 7 und 6<br>83,2 | C 54,48<br>H 6,72<br>N 16,03 | 54,27<br>6,67<br>15,82 | Gly<br>1,00 | Leu<br>1,01 | Arg<br>1,00 |
| 69 | $CH_3SO_2$-D-Leu-Gly-Arg-MCA · AcOH<br>$C_{27}H_{41}N_7O_9S$ | 68f (5 mmol)<br>$CH_3SO_2$-Cl<br>(5,5 mmol) | Bsp. 7 und 6<br>78,4 | C 50,93<br>H 6,51<br>N 15,53<br>S 4,92 | 50,69<br>6,46<br>15,33<br>5,01 | Gly<br>1,00 | Leu<br>0,99 | Arg<br>0,98 |
| 70 | Cbo-D-Leu-Gly-Arg-5-$NO_2$-1-NA · HBr<br>$C_{32}H_{41}N_8O_7Br$ | 70d (5 mmol)<br>Cbo-D-Leu-OpNP<br>(5,5 mmol) | Bsp. 1e<br>75,8 | C 52,88<br>H 5,69<br>N 15,53<br>Br 10,82 | 52,68<br>5,66<br>15,36<br>10,95 | Gly<br>1,00 | Leu<br>1,01 | Arg<br>0,99 |
| 71 | $CH_3SO_2$-D-Leu-Gly-Arg-5-$NO_2$-1-NA · AcOH<br>$C_{27}H_{40}N_8O_9S$ | 70f (5 mmol)<br>$CH_3SO_2$-Cl<br>(5,5 mmol) | Bsp. 7 und 6<br>80,8 | C 50,08<br>H 6,24<br>N 17,29<br>S 4,82 | 49,68<br>6,18<br>17,17<br>4,91 | Gly<br>1,00 | Leu<br>1,00 | Arg<br>0,97 |
| 72 | Cbo-D-Leu-Gly-Arg-5-ChA · HBr<br>$C_{31}H_{41}N_8O_5Br$ | 72d (5 mmol)<br>Cbo-D-Leu-OpNP<br>(5,5 mmol) | Bsp. 1e<br>78,5 | C 54,28<br>H 6,07<br>N 16,48<br>Br 11,54 | 54,31<br>6,03<br>16,34<br>11,65 | Gly<br>1,00 | Leu<br>0,98 | Arg<br>0,98 |
| 73 | $CH_3SO_2$-D-Leu-Gly-Arg-5-ChA · AcOH<br>$C_{26}H_{40}N_7O_4S$ | 72f (5 mmol)<br>$CH_3SO_2$-Cl<br>(5,5 mmol) | Bsp. 7 und 6<br>76,5 | C 51,68<br>H 6,67<br>N 18,52<br>S 5,18 | 51,30<br>6,62<br>18,41<br>5,27 | Gly<br>1,00 | Leu<br>0,99 | Arg<br>0,98 |
| 74 | Cbo-D-Leu-Gly-Arg-8-$NO_2$-5-ChA · HBr<br>$C_{31}H_{40}N_9O_7Br$ | 72d (5 mmol)<br>Cbo-D-Leu-OpNP<br>(5,5 mmol) | Bsp. 1e<br>79,0 | C 51,11<br>H 5,54<br>N 17,48<br>Br 10,76 | 50,96<br>5,52<br>17,25<br>10,94 | Gly<br>1,00 | Leu<br>0,98 | Arg<br>0,97 |
| 75 | $CH_3SO_2$-D-Leu-Gly-Arg-8-$NO_2$-5-ChA · AcOH<br>$C_{26}H_{39}N_9O_9S$ | 74f (5 mmol)<br>$CH_3SO_2$-Cl<br>(5,5 mmol) | Bsp. 7 und 6<br>75,7 | C 48,03<br>H 6,07<br>N 19,44<br>S 4,83 | 47,77<br>6,01<br>19,28<br>4,91 | Gly<br>1,00 | Leu<br>0,98 | Arg<br>0,97 |
| 76 | BOC-D-Nval-But-Arg-pNA · HBr<br>$C_{26}H_{43}N_8O_7Br$ | 76d (5 mmol)<br>BOC-D-Nval-OpNP<br>(5,5 mmol) | Bsp. 1e<br>74,6 | C 47,45<br>H 6,61<br>N 17,24<br>Br 11,88 | 47,34<br>6,57<br>16,99<br>12,11 | Nval<br>1,00 | But<br>0,98 | Arg<br>0,98 |
| 77 | BOC-D-Nval-N(n-Bu)Gly-Arg-pNA · HBr<br>$C_{28}H_{47}N_8O_7Br$ | 77d (5 mmol)<br>BOC-D-Nval-OpNP<br>(5,5 mmol) | Bsp. 1e<br>66,3 | C 49,08<br>H 6,95<br>N 16,53<br>Br 11,41 | 48,91<br>6,89<br>16,30<br>11,62 | Nval<br>1,00 | Arg<br>0,98 | |

Tabelle 1 *(Fortsetzung)*

| Bsp. | Endprodukt | Ausgangs-produkte (mmol) | Methode Aus-beute (%) | Elementaranalyse | gef. (%) | ber. (%) | Aminosäureanalyse | | |
|---|---|---|---|---|---|---|---|---|---|
| 78 | β-Naphthyl-$SO_2$-<br>D-Leu-Gly-Arg-<br>pNA · HBr<br>$C_{30}H_{39}N_8O_7SBr$ | 7f (5 mmol)<br>β-NA-$SO_2$-Cl<br>(5,5 mmol) | Bsp. 7<br>80,2 | C<br>H<br>N<br>Br<br>S | 49,25<br>5,41<br>15,43<br>10,70<br>4,15 | 48,98<br>5,34<br>15,23<br>10,86<br>4,36 | Gly<br>1,00 | Leu<br>0,98 | Arg<br>0,97 |
| 79 | 4-$NH_2$-Bz-D-<br>Nleu-Gly-Arg-<br>pNA · 2HBr<br>$C_{27}H_{39}N_9O_6Br_2$ | 34f (5 mmol)<br>(HCl)p-$NH_2$-Bz-<br>Cl<br>(5,5 mmol) | Bsp. 7<br>und 6<br>65,0 | C<br>H<br>N<br>Br | 43,68<br>5,32<br>17,13<br>21,05 | 43,50<br>5,27<br>16,91<br>21,44 | Gly<br>1,00 | Nleu<br>1,02 | Arg<br>0,98 |
| 80 | 4-Me-Cbo-D-Leu-<br>Gly-Arg-pNA ·<br>AcOH<br>$C_{31}H_{44}N_8O_9$ | 1d (5 mmol)<br>4-Me-Cbo-D-<br>Leu-OpNP<br>(5,5 mmol) | Bsp. 7<br>und 6<br>75,0 | C<br>H<br>N | 55,68<br>6,64<br>16,85 | 55,35<br>6,59<br>16,66 | Gly<br>1,00 | Leu<br>1,01 | Arg<br>0,99 |
| 81 | 4-Cl-Cbo-D-Leu-<br>Gly-Arg-pNA ·<br>AcOH<br>$C_{30}H_{41}N_8O_9Cl$ | 1d (5 mmol)<br>4-Cl-Cbo-D-Leu-<br>OpNP<br>(5,5 mmol) | Bsp. 1e<br>und 6<br>69,3 | C<br>H<br>N<br>Cl | 52,18<br>5,99<br>16,25<br>5,03 | 51,98<br>5,96<br>16,17<br>5,12 | Gly<br>1,00 | Leu<br>1,01 | Arg<br>0,98 |
| 82 | BOC-D-(α)-AOA-<br>Gly-Arg-pNA ·<br>AcOH<br>$C_{29}H_{48}N_8O_9$ | 1d (5 mmol)<br>BOC-D-(α)-<br>AOA-OpNP<br>(5,5 mmol) | Bsp. 1e<br>und 6<br>65,4 | C<br>H<br>N | 53,45<br>7,51<br>17,38 | 53,36<br>7,41<br>17,17 | Gly<br>1,00 | Arg<br>0,99 | |
| 83 | Cbo-D-Tyr(OBzl)-<br>Gly-Arg-pNA · HBr<br>$C_{38}H_{43}N_8O_8Br$ | 1d (5 mmol)<br>Cbo-D-Tyr-<br>(OBzl)-OpNP<br>(5,5 mmol) | Bsp. 1e<br>75,5 | C<br>H<br>N<br>Br | 55,26<br>5,33<br>13,85<br>9,58 | 5,68<br>5,29<br>13,67<br>9,75 | Gly<br>1,00 | Tyr<br>0,97 | Arg<br>0,99 |
| 84 | $CH_3O$-CO-D-Tyr-<br>Gly-Arg-pNA ·<br>AcOH<br>$C_{27}H_{36}N_8O_{10}$ | 83f (5 mmol)<br>$CH_3O$-CO-Cl<br>(5,5 mmol) | Bsp. 7<br>und 6<br>58,4 | C<br>H<br>N | 51,09<br>5,77<br>17,90 | 51,26<br>5,74<br>17,71 | Gly<br>1,00 | Tyr<br>0,98 | Arg<br>1,01 |
| 85 | Cbo-D-Ser-<br>(OtBu)-Gly-Arg-<br>pNA · AcOH<br>$C_{31}H_{44}N_8O_{10}$ | 1d (5 mmol)<br>Cbo-D-Ser-<br>(OtBu)-OpNP<br>(5,5 mmol) | Bsp. 1e<br>und 6<br>73,8 | C<br>H<br>N | 53,78<br>6,48<br>16,45 | 54,06<br>6,44<br>16,27 | Gly<br>1,00 | Ser<br>0,96 | Arg<br>0,99 |
| 86 | Cbo-D-Ser-Gly-<br>Arg-pNA · AcOH<br>$C_{27}H_{36}N_8O_{10}$ | 85 (5 mmol)<br>$CF_3COOH$<br>(10 mmol) | Beispiele<br>1d & 6<br>85,3 | C<br>H<br>N | 41,00<br>5,79<br>18,02 | 51,26<br>5,74<br>17,71 | Gly<br>1,00 | Ser<br>0,97 | Arg<br>1,00 |
| 87 | Cbo-D-Ser(OMe)-<br>Gly-Arg-pNA ·<br>AcOH<br>$C_{28}H_{38}N_8O_{10}$ | 1d (5 mmol)<br>Cbo-D-Ser-<br>(OMe)-OpNP<br>(5,5 mmol) | Bsp. 1e<br>und 6<br>74,6 | C<br>H<br>N | 51,75<br>6,00<br>17,47 | 52,01<br>5,92<br>17,33 | Gly<br>1,00 | Arg<br>0,98 | |
| 88 | Cbo-D-Ser(OBzl)-<br>Gly-Arg-pNA ·<br>AcOH<br>$C_{34}H_{42}N_8O_{10}$ | 1d (5 mmol)<br>Cbo-D-Ser-<br>(OBzl)-OpNP<br>(5,5 mmol) | Bsp. 1e<br>und 6<br>80,4 | C<br>H<br>N | 55,95<br>5,91<br>15,68 | 56,50<br>5,86<br>15,50 | Gly<br>1,00 | Arg<br>0,99 | |

Tabelle 1 *(Fortsetzung)*

| Bsp. | Endprodukt | Ausgangs-produkte (mmol) | Methode Aus-beute (%) | Elementaranalyse gef. (%) | ber. (%) | Aminosäureanalyse | | |
|---|---|---|---|---|---|---|---|---|
| 89 | Cbo-D-Thr-(OtBu)-Gly-Arg-pNA · AcOH $C_{32}H_{46}N_8O_{10}$ | 1d (5 mmol) Cbo-D-Thr-(OtBu)-OpNP (5,5 mmol) | Bsp. 1e und 6 78,4 | C 54,19<br>H 6,66<br>N 16,17 | 54,69<br>6,60<br>15,95 | Gly 1,00 | Thr 0,96 | Arg 0,98 |
| 90 | Cbo-D-Thr-Gly-Arg-pNA · AcOH $C_{28}H_{38}N_8O_{10}$ | 89 (5 mmol) $CF_3COOH$ (10 mmol) | Beispiele 1d & 6 82,0 | C 51,88<br>H 5,95<br>N 17,45 | 52,01<br>5,92<br>17,33 | Gly 1,00 | Thr 0,97 | Arg 0,98 |
| 91 | Cbo-D-Thr(OMe)-Gly-Arg-pNA · AcOH $C_{29}H_{40}N_8O_{10}$ | 1d (5 mmol) Cbo-D-Thr-(OMe)-OpNP (5,5 mmol) | Bsp. 1e und 6 75,5 | C 52,15<br>H 6,13<br>N 17,20 | 52,72<br>6,10<br>16,96 | Gly 1,00 | Arg 0,98 | |
| 92 | Cbo-D-Thr(OBzl)-Gly-Arg-pNA · AcOH $C_{35}H_{44}N_8O_{10}$ | 1d (5 mmol) Cbo-D-Thr-(OBzl)-OpNP (5,5 mmol) | Bsp. 1e und 6 81,2 | C 56,50<br>H 6,07<br>N 15,45 | 57,06<br>6,02<br>15,21 | Gly 1,00 | Arg 1,00 | |
| 93 | Cbo-D-Asp-Gly-Arg-pNA $C_{26}H_{32}N_8O_9$ | 18 (5 mmol) $CF_3COOH$ (15 mmol) | Beispiele 1d & 6 85,0 | C 51,66<br>H 5,43<br>N 18,95 | 52,00<br>5,37<br>18,66 | Gly 1,00 | Asp 0,98 | Arg 1,01 |
| 94 | Cbo-D-Glu-Gly-Arg-pNA $C_{27}H_{34}N_8O_9$ | 17 (5 mmol) $CF_3COOH$ (15 mmol) | Beispiele 1d & 6 82,4 | C 52,21<br>H 5,61<br>N 18,48 | 52,76<br>5,58<br>18,23 | Gly 1,00 | Glu 0,97 | Arg 0,99 |
| 95 | Cbo-D-Glu(γ-OMe)-Gly-Arg-pNA · AcOH $C_{30}H_{40}N_8O_{11}$ | 1d (5 mmol) Cbo-D-Glu(γ-OMe)-OpNP (5,5 mmol) | Bsp. 1e und 6 78,6 | C 51,84<br>H 5,89<br>N 16,47 | 52,32<br>5,85<br>16,27 | Gly 1,00 | Glu 1,01 | Arg 0,98 |
| 96 | Cbo-D-Asp(β-OMe)-Gly-Arg-pNA · AcOH $C_{29}H_{38}N_8O_{11}$ | 1d (5 mmol) Cbo-D-Asp(β-OMe)-OpNP (5,5 mmol) | Bsp. 1e und 6 78,9 | C 51,48<br>H 5,71<br>N 16,93 | 51,63<br>5,68<br>16,61 | Gly 1,00 | Asp 1,02 | Arg 1,00 |
| 97 | Cbo-D-Glu(γ-OBzl)-Gly-Arg-pNA · AcOH $C_{35}H_{42}N_8O_{11}$ | 1d (5 mmol) Cbo-D-Glu(γ-OBzl)-OpNP (5,5 mmol) | Bsp. 1e und 6 76,7 | C 55,38<br>H 5,68<br>N 15,09 | 55,99<br>5,64<br>14,93 | Gly 1,00 | Glu 0,98 | Arg 0,99 |
| 98 | Cbo-D-Asp(β-OBzl)-Gly-Arg-pNA · AcOH $C_{34}H_{40}N_8O_{11}$ | 1d (5 mmol) Cbo-D-Asp(β-OBzl)-OpNP (5,5 mmol) | Bsp. 1e und 6 74,8 | C 55,03<br>H 5,51<br>N 15,48 | 55,43<br>5,47<br>15,21 | Gly 1,00 | Asp 0,99 | Arg 0,97 |
| 99 | Cbo-D-Aadi(δ-OtBu)-Gly-Arg-pNA · AcOH $C_{34}H_{48}N_8O_{11}$ | 1d (5 mmol) Cbo-D-Aadi(δ-OtBu)-OpNP (5,5 mmol) | Bsp. 1e und 6 71,5 | C 54,13<br>H 6,57<br>N 15,29 | 54,83<br>6,50<br>15,05 | Gly 1,00 | Arg 0,98 | |
| 100 | Cbo-D-Aadi-Gly-Arg-pNA $C_{28}H_{36}N_8O_9$ | 99 (5 mmol) $CF_3COOH$ (15 mmol) | Beispiele 1d & 6 81,1 | C 52,97<br>H 5,83<br>N 18,05 | 53,50<br>5,77<br>17,83 | Gly 1,00 | Arg 0,99 | |

Tabelle 1 *(Fortsetzung)*

| Bsp. | Endprodukt | Ausgangs-produkte (mmol) | Methode Aus-beute (%) | Elementaranalyse gef. (%) | Elementaranalyse ber. (%) | Aminosäureanalyse | | |
|---|---|---|---|---|---|---|---|---|
| 101 | Cbo-D-Aadi(δ-OBzl)-Gly-Arg-pNA · AcOH $C_{37}H_{46}N_8O_{11}$ | 1d (5 mmol) Cbo-D-Aadi(δ-OBzl)-OpNP (5,5 mmol) | Bsp. 1e und 6 73,9 | C 56,53<br>H 6,00<br>N 14,65 | 57,06<br>5,95<br>14,39 | Gly 1,00 | Arg 0,99 | |

Tabelle 2

| Beispiel | Aminosäure oder Dipeptidzwischenprodukt | Ausgangsprodukt (mmol) | Methode Ausbeute (%) | Elementaranalyse gef. (%) | Elementaranalyse ber. (%) |
|---|---|---|---|---|---|
| 20c | Cbo-Sar-Arg-pNA · HBr $C_{23}H_{30}N_7O_6Br$ | 1b (10 mmol) Cbo-Sar-OpNP (11 mmol) | Beispiel 1c 84,1 | C 47,88<br>H 5,25<br>N 17,08<br>Br 13,50 | 47,59<br>5,21<br>16,89<br>13,77 |
| 20d | 2HBr · H-Sar-Arg-pNA $C_{15}H_{25}N_7O_4Br_2$ | 20c (7 mmol) 2N HBr/AcOH | Beispiel 1d 91,2 | C 34,50<br>H 4,82<br>N 18,91<br>Br 30,06 | 34,17<br>4,78<br>18,60<br>30,31 |
| 21c | Cbo-Ala-Arg-pNA · HBr $C_{23}H_{30}N_7O_6Br$ | 1b (10 mmol) Cbo-Ala-OpNP (11 mmol) | Beispiel 1c 83,6 | C 47,69<br>H 5,28<br>N 16,95<br>Br 13,46 | 47,59<br>5,21<br>16,89<br>13,77 |
| 21d | 2HBr · H-Ala-Arg-pNA $C_{15}H_{25}N_7O_4Br_2$ | 21c (7 mmol) 2N HBr/AcOH | Beispiel 1d 93,6 | C 34,28<br>H 4,79<br>N 18,90<br>Br 29,88 | 34,17<br>4,78<br>18,60<br>30,31 |
| 24c | Cbo-N(Et)Gly-Arg-pNA · HBr $C_{24}H_{32}N_7O_6Br$ | 1b (10 mmol) Cbo-N(Et)Gly-OpNP (11 mmol) | Beispiel 1c 86,0 | C 48,62<br>H 5,48<br>N 16,55<br>Br 13,25 | 48,49<br>5,43<br>16,49<br>13,44 |
| 24d | 2HBr · H-N(Et)Gly-Arg-pNA $C_{16}H_{27}N_7O_4Br_2$ | 24c (7 mmol) 2N HBr/AcOH | Beispiel 1d 94,0 | C 35,70<br>H 5,09<br>N 18,30<br>Br 29,22 | 35,51<br>5,03<br>18,12<br>29,53 |
| 25c | Cbo-N(Pr)Gly-Arg-pNA · HBr $C_{25}H_{34}N_7O_6Br$ | 1b (10 mmol) Cbo-N(Pr)Gly-OpNP (11 mmol) | Beispiel 1c 82,5 | C 50,08<br>H 5,69<br>N 16,36<br>Br 12,88 | 49,35<br>5,63<br>16,11<br>13,13 |
| 25d | 2HBr · H-N(Pr)Gly-Arg-pNA $C_{17}H_{29}N_7O_4Br_2$ | 25c (7 mmol) 2N HBr/AcOH | Beispiel 1d 88,5 | C 36,68<br>H 5,31<br>N 17,96<br>Br 28,58 | 36,77<br>5,26<br>17,66<br>28,78 |

Tabelle 2 *(Fortsetzung)*

| Beispiel | Aminosäure oder Dipeptidzwischenprodukt | Ausgangsprodukt (mmol) | Methode Ausbeute (%) | Elementaranalyse gef. (%) | ber. (%) |
|---|---|---|---|---|---|
| 60c | Cbo-Gly-Arg-2-NA · HBr<br>$C_{26}H_{31}N_6O_4Br$ | 4b (5 mmol)<br>Cbo-Gly-OpNP<br>(5,5 mmol) | Beispiel 4c<br>75,6 | C 54,24<br>H 5,52<br>N 14,95<br>Br 13,75 | 54,64<br>5,47<br>14,71<br>13,98 |
| 60d | 2HBr · H-Gly-Arg-2-NA<br>$C_{18}H_{26}N_6O_2Br_2$ | 60c (5 mmol)<br>2N HBr/AcOH | Beispiel 4d<br>90,6 | C 41,50<br>H 5,10<br>N 16,62<br>Br 30,15 | 41,72<br>5,06<br>16,22<br>30,84 |
| 63c | Cbo-Gly-Arg-4-MeO-2-NA · HBr<br>$C_{27}H_{33}N_6O_5Br$ | 5b (5 mmol)<br>Cbo-Gly-OpNP<br>(5,5 mmol) | Beispiel 5c<br>88,4 | C 53,41<br>H 5,55<br>N 14,22<br>Br 12,95 | 53,91<br>5,53<br>13,97<br>13,28 |
| 63d | 2HBr · H-Gly-Arg-4-MeO-2-NA<br>$C_{19}H_{28}N_6O_3Br_2$ | 63c (5 mmol)<br>2N HBr/AcOH | Beispiel 5d<br>90,9 | C 41,32<br>H 5,19<br>N 15,54<br>Br 28,85 | 41,62<br>5,15<br>15,33<br>29,15 |
| 70a | Cbo-Arg-5-$NO_2$-1-NA · HCl<br>$C_{24}H_{27}N_6O_5Cl$ | Cbo-Arg-OH · HCl<br>(25 mmol)<br>5-$NO_2$-1-naphthylamin<br>(25 mmol) | Beispiel 3a<br>38,7 | C 55,65<br>H 5,31<br>N 16,54<br>Cl 6,77 | 55,98<br>5,28<br>16,32<br>6,89 |
| 70b | 2HBr · H-Arg-5-$NO_2$-1-NA<br>$C_{16}H_{22}N_6O_3Br_2$ | 70a (5 mmol)<br>2N Hbr/AcOH | Beispiel 4b<br>88,5 | C 37,85<br>H 4,41<br>N 16,78<br>Br 31,10 | 37,96<br>4,38<br>16,60<br>31,57 |
| 70c | Cbo-Gly-Arg-5-$NO_2$-1-NA · HBr<br>$C_{26}H_{30}N_7O_6Br$ | 70b (3 mmol)<br>Cbo-Gly-OpNP<br>(3,3 mmol) | Beispiel 4c<br>78,6 | C 50,25<br>H 4,99<br>N 16,10<br>Br 12,76 | 50,66<br>4,91<br>15,91<br>12,96 |
| 70d | 2HBr · H-Gly-Arg-5-$NO_2$-1-NA<br>$C_{18}H_{25}N_7O_4Br_2$ | 70c (2,5 mmol)<br>2N HBr/AcOH | Beispiel 1d<br>91 | C 38,17<br>H 4,51<br>N 17,63<br>Br 28,00 | 38,38<br>4,47<br>17,41<br>28,37 |
| 72a | Cbo-Arg-5-ChA · HCl<br>$C_{23}H_{27}N_6O_3Cl$ | Cbo-Arg-OH · HCl<br>(25 mmol)<br>5-Aminochinolin<br>(25 mmol) | Beispiel 3a<br>31,9 | C 58,09<br>H 5,84<br>N 18,06<br>Cl 7,44 | 58,66<br>5,78<br>17,85<br>7,53 |
| 72b | 2HBr · H-Arg-5-ChA<br>$C_{15}H_{22}N_6OBr_2$ | 72a (5 mmol)<br>2N HBr/AcOH | Beispiel 1d<br>81,8 | C 38,75<br>H 4,83<br>N 18,45<br>Br 33,88 | 38,98<br>4,80<br>18,18<br>34,58 |
| 72c | Cbo-Gly-Arg-5-ChA · HBr<br>$C_{25}H_{30}N_7O_4Br$ | 72b (3 mmol)<br>Cbo-Gly-OpNP<br>(3,3 mmol) | Beispiel 4c<br>68,5 | C 52,17<br>H 5,32<br>N 17,17<br>Br 13,63 | 52,45<br>5,28<br>17,13<br>13,96 |

Tabelle 2 *(Fortsetzung)*

| Beispiel | Aminosäure oder Dipeptidzwischenprodukt | Ausgangsprodukt (mmol) | Methode Ausbeute (%) | Elementaranalyse gef. (%) | ber. (%) |
|---|---|---|---|---|---|
| 72d | 2HBr · H-Gly-Arg-5-ChA<br>$C_{17}H_{26}N_7O_2Br_2$ | 72c (2,5 mmol)<br>2N HBr/AcOH | Beispiel 1d<br>78,3 | C 39,19<br>H 4,90<br>N 19,11<br>Br 30,45 | 39,92<br>4,85<br>18,88<br>30,78 |
| 74a | Cbo-Arg-8-$NO_2$-5-ChA · HCl<br>$C_{23}H_{26}N_7O_5Cl$ | Cbo-Arg-OH · HCl<br>(25 mmol)<br>5-$NH_2$-8-$NO_2$-chinolin<br>(25 mmol) | Beispiel 3a<br>33,0 | C 53,14<br>H 5,11<br>N 19,21<br>Cl 6,80 | 53,54<br>5,08<br>19,00<br>6,87 |
| 74b | 2HBr · H-Arg-8-$NO_2$-5-ChA<br>$C_{15}H_{21}N_7O_3Br_2$ | 74a (5 mmol)<br>2N Hbr/AcOH | Beispiel 1d<br>84,2 | C 35,42<br>H 4,20<br>N 19,50<br>Br 30,99 | 35,52<br>4,17<br>19,33<br>31,51 |
| 74c | Cbo-Gly-Arg-8-$NO_2$-5-ChA · HBr<br>$C_{25}H_{29}N_8O_6Br$ | 74b (3 mmol)<br>Cbo-Gly-OpNP<br>(3,3 mmol) | Beispiel 4c<br>68,5 | C 48,32<br>H 4,77<br>N 18,28<br>Br 12,74 | 48,63<br>4,73<br>18,15<br>12,94 |
| 74d | 2HBr · H-Gly-Arg-8-$NO_2$-5-ChA<br>$C_{17}H_{24}N_8O_4Br_2$ | 74c (2,5 mmol)<br>2N HBr/AcOH | Beispiel 1d<br>76,9 | C 36,00<br>H 4,33<br>N 20,05<br>Br 27,95 | 36,19<br>4,29<br>19,86<br>28,32 |
| 76c | Cbo-But-Arg-pNA · HBr<br>$C_{24}H_{32}N_7O_6Br$ | 1b (5 mmol)<br>Cbo-But-OpNP<br>(5,5 mmol) | Beispiel 1c<br>80,4 | C 48,55<br>H 5,47<br>N 16,75<br>Br 13,20 | 48,49<br>5,43<br>16,49<br>13,44 |
| 76d | 2HBr · H-But-Arg-pNA<br>$C_{16}H_{27}N_7O_4Br_2$ | 76c (3 mmol)<br>2N HBr/AcOH | Beispiel 1d<br>84,8 | C 35,27<br>H 5,03<br>N 18,38<br>Br 29,25 | 35,51<br>5,03<br>18,12<br>29,53 |
| 77c | Cbo-N(n-Bu)Gly-Arg-pNA · HBr<br>$C_{26}H_{36}N_7O_6Br$ | 1b (5 mmol)<br>Cbo-N(n-Bu)Gly-OpNP<br>(5,5 mmol) | Beispiel 1c<br>68,2 | C 49,75<br>H 5,85<br>N 15,90<br>Br 12,63 | 50,16<br>5,83<br>15,75<br>12,84 |
| 77d | 2HBr · H-N(n-Bu)Gly-Arg-pNA<br>$C_{18}H_{31}N_7O_4Br_2$ | 77c (2,5 mmol)<br>2N HBr/AcOH | Beispiel 1d<br>80,7 | C 37,88<br>H 5,54<br>N 17,50<br>Br 27,70 | 37,97<br>5,49<br>17,22<br>28,07 |

Tabelle 3

| Beispiel | Tripeptidzwischenprodukt | Ausgangsprodukte (mmol) | Methode Ausbeute (%) | Elementaranalyse | gef. (%) | ber. (%) |
|---|---|---|---|---|---|---|
| 7f | 2HBr · H-D-Leu-Gly-Arg-pNA $C_{20}H_{34}N_8O_5Br_2$ | 1 (5 mmol) 2N HBr/AcOH | Beispiel 2d 96,3 | C<br>H<br>N<br>Br | 38,09<br>5,41<br>17,95<br>25,42 | 38,35<br>5,47<br>17,89<br>25,51 |
| 34f | 2HBr · H-D-Nleu-Gly-Arg-pNA $C_{20}H_{34}N_8O_5Br_2$ | 8 (5 mmol) 2N HBr/AcOH | Beispiel 2d 93,4 | C<br>H<br>N<br>Br | 38,18<br>5,52<br>18,06<br>25,20 | 38,35<br>5,47<br>17,89<br>25,51 |
| 37f | 2HBr · H-D-Leu-Sar-Arg-pNA $C_{21}H_{36}N_8O_5Br_2$ | 20 (5 mmol) 2N HBr/AcOH | Beispiel 2d 92,1 | C<br>H<br>N<br>Br | 39,15<br>5,70<br>17,81<br>24,81 | 39,39<br>5,67<br>17,50<br>24,96 |
| 42f | 2HBr · H-D-Nleu-Sar-Arg-pNA $C_{21}H_{36}N_8O_5Br_2$ | 28 (5 mmol) 2N HBr/AcOH | Beispiel 2d 96,2 | C<br>H<br>N<br>Br | 39,08<br>5,63<br>17,75<br>24,70 | 39,39<br>5,67<br>17,50<br>24,96 |
| 48f | 2HBr · H-D-Nval-Gly-Arg-pNA $C_{19}H_{32}N_8O_5Br_2$ | 9 (5 mmol) 2N HBr/AcOH | Beispiel 2d 91,8 | C<br>H<br>N<br>Br | 37,02<br>5,33<br>18,55<br>25,88 | 37,27<br>5,27<br>18,30<br>26,10 |
| 53f | 2HBr · H-D-Nval-Sar-Arg-pNA $C_{20}H_{34}N_8O_5Br_2$ | 29 (5 mmol) 2N HBr/AcOH | Beispiel 2d 95,5 | C<br>H<br>N<br>Br | 38,38<br>5,54<br>18,10<br>25,25 | 38,35<br>5,47<br>17,89<br>25,51 |
| 57f | 2HBr · H-D-CHA-Gly-Arg-pNA $C_{23}H_{38}N_8O_5Br_2$ | 12 (5 mmol) 2N HBr/AcOH | Beispiel 1d 82,3 | C<br>H<br>N<br>Br | 41,50<br>5,77<br>17,07<br>23,71 | 41,45<br>5,75<br>16,82<br>23,98 |
| 60f | 2HBr · H-D-Leu-Gly-Arg-2-NA $C_{24}H_{36}N_7O_3Br_2$ | 60 (5 mmol) 2N HBr/AcOH | Beispiel 1d 78,5 | C<br>H<br>N<br>Br | 45,43<br>5,81<br>15,81<br>25,05 | 45,73<br>5,76<br>15,55<br>25,35 |
| 63f | 2HBr · H-D-Leu-Gly-Arg-4-MeO-2-NA $C_{25}H_{38}N_7O_4Br_2$ | 63 (5 mmol) 2N HBr/AcOH | Beispiel 1d 76,9 | C<br>H<br>N<br>Br | 45,18<br>5,83<br>15,03<br>23,85 | 45,47<br>5,80<br>14,85<br>24,20 |
| 66f | 2HBr · H-D-Leu-Gly-Arg-DPA $C_{24}H_{38}N_7O_7Br_2$ | 3e (5 mmol) 2N HBr/AcOH | Beispiel 1d 76,9 | C<br>H<br>N<br>Br | 41,20<br>5,55<br>14,23<br>22,66 | 41,39<br>5,50<br>14,08<br>22,95 |
| 68f | 2HBr · H-D-Leu-Gly-Arg-MCA $C_{24}H_{36}N_7O_5Br_2$ | 2e (5 mmol) 2N HBr/AcOH | Beispiel 1d 80,8 | C<br>H<br>N<br>Br | 43,17<br>5,51<br>15,00<br>23,81 | 43,52<br>5,48<br>14,80<br>24,13 |

Tabelle 3 *(Fortsetzung)*

| Beispiel | Tripeptidzwischenprodukt | Ausgangsprodukte (mmol) | Methode Ausbeute (%) | Elementaranalyse gef. (%) | ber. (%) |
|---|---|---|---|---|---|
| 70f | 2HBr · H-D-Leu-Gly-Arg-5-$NO_2$-1-NA $C_{24}H_{35}N_8O_5Br_2$ | 70 (5 mmol) 2N HBr/AcOH | Beispiel 1d 68,4 | C 42,51<br>H 5,24<br>N 16,83<br>Br 23,45 | 42,68<br>5,22<br>16,59<br>23,66 |
| 72f | 2HBr · H-D-Leu-Gly-Arg-5-ChA $C_{23}H_{35}N_8O_3Br_2$ | 72 (5 mmol) 2N HBr/AcOH | Beispeil 1d 75,3 | C 43,68<br>H 5,61<br>N 17,95<br>Br 25,08 | 43,75<br>5,59<br>17,75<br>25,31 |
| 74f | 2HBr · H-D-Leu-Gly-Arg-8-$NO_2$-5-ChA $C_{23}H_{34}N_9O_5Br_2$ | 74 (5 mmol) 2N HBr/AcOH | Beispiel 1d 72,7 | C 40,75<br>H 5,10<br>N 18,95<br>Br 23,30 | 40,84<br>5,07<br>18,64<br>23,63 |
| 83f | 2($CF_3COOH$) · H-Tyr-Gly-Arg-pNA $C_{27}H_{32}N_8O_{10}F_6$ | 83 (5 mmol) $H_2F_2$ (10 ml) | Beispiele 1d und 6 63,8 | C 43,37<br>H 4,35<br>N 15,24<br>F 14,88 | 43,67<br>4,34<br>15,09<br>15,35 |

Die Empfindlichkeit der erfindungsgemässen Tripeptidderivate gegenüber Faktor Xa ist in der nachfolgenden Tabelle 4 zahlenmässig angegeben und mit derjenigen des vorbekannten Substrates Bz-Ile-Glu(γ-OH)-Gly-Arg-pNA · HCl verglichen.

Tabelle 4

Aktivität von 1 ml wässeriger Lösung von bovinem Faktor Xa und von durch Aktivierung von Faktor X in 1 ml menschlichem Normalplasma mittels RVV erzeugtem Faktor Xa, ausgedrückt in Nanomol des pro Minute freigesetzten Spaltproduktes $H-R^5$.

| Substrat-konzentration ($2\times10^{-4}$) | Boviner Faktor (Xa) | Humaner Faktor (Xa) |
|---|---|---|
| Substrate gemäss Beispielen | | |
| 1 | 2560 | 1520 |
| 2 | 1180 | 760 |
| 3 | 1405 | 985 |
| 4 | 1290 | 895 |
| 5 | 1140 | 815 |
| 6 | 2550 | 1520 |
| 7 | 950 | 705 |
| 8 | 2780 | 1568 |
| 9 | 2010 | 1105 |
| 10 | 1332 | 825 |
| 11 | 246 | 100 |
| 12 | 1960 | 1528 |
| 13 | 1936 | 1548 |
| 14 | 1436 | 1468 |
| 15 | 1630 | 1320 |
| 16 | 1385 | 1235 |

Tabelle 4 *(Fortsetzung)*

| Substrat-konzentration ($2\times10^{-4}$) | Boviner Faktor (Xa) | Humaner Faktor (Xa) |
|---|---|---|
| 17 | 1290 | 660 |
| 18 | 1045 | 595 |
| 19 | 1675 | 795 |
| 20 | 1555 | 1432 |
| 21 | 985 | 580 |
| 22 | 2214 | 1000 |
| 23 | 2280 | 820 |
| 24 | 1205 | 495 |
| 25 | 1025 | 435 |
| 26 | 2028 | 1656 |
| 27 | 1940 | 1840 |
| 28 | 1986 | 1608 |
| 29 | 1160 | 1016 |
| 30 | 1070 | 695 |
| 31 | 1560 | 987 |
| 32 | 1040 | 560 |
| 33 | 1380 | 890 |
| 34 | 210 | 105 |
| 35 | 280 | 115 |
| 36 | 295 | 135 |
| 37 | 980 | 620 |
| 38 | 825 | 570 |
| 39 | 600 | 420 |
| 40 | 30 | 20 |
| 41 | 60 | 30 |
| 42 | 105 | 75 |
| 43 | 250 | 112 |
| 44 | 205 | 108 |
| 45 | 380 | 206 |
| 46 | 180 | 130 |
| 47 | 410 | 218 |

Tabelle 4 *(Fortsetzung)*

| Substrat-konzentration ($2 \times 10^{-4}$) | Boviner Faktor (Xa) | Humaner Faktor (Xa) |
|---|---|---|
| 48 | 550 | 420 |
| 49 | 560 | 425 |
| 50 | 555 | 415 |
| 51 | 680 | 510 |
| 52 | 740 | 505 |
| 53 | 980 | 1200 |
| 54 | 850 | 1060 |
| 55 | 2440 | 1360 |
| 56 | 2540 | 1430 |
| 57 | 2260 | 1260 |
| 58 | 3230 | 1420 |
| 59 | 2660 | 1770 |
| 60 | 1420 | 995 |
| 61 | 1540 | 1065 |
| 62 | 1520 | 985 |
| 63 | 1280 | 1025 |
| 64 | 1370 | 965 |
| 65 | 1410 | 1005 |
| 66 | 1490 | 1010 |
| 67 | 1515 | 1030 |
| 68 | 1235 | 795 |
| 69 | 1280 | 810 |
| 70 | 1180 | 830 |
| 71 | 1265 | 875 |
| 72 | 1005 | 660 |
| 73 | 1145 | 745 |
| 74 | 965 | 715 |
| 75 | 1080 | 735 |
| 76 | 795 | 440 |
| 77 | 850 | 360 |
| 78 | 940 | 635 |
| 79 | 495 | 385 |
| 80 | 2530 | 1380 |
| 81 | 2480 | 1365 |
| 82 | 2210 | 1690 |
| 83 | 690 | 580 |
| 84 | 890 | 610 |
| 85 | 330 | 146 |
| 86 | 360 | 165 |
| 87 | 420 | 170 |
| 88 | 510 | 225 |
| 89 | 915 | 830 |
| 90 | 610 | 415 |
| 91 | 780 | 630 |
| 92 | 1020 | 845 |
| 93 | 750 | 465 |
| 94 | 830 | 505 |
| 95 | 1880 | 1420 |
| 96 | 1230 | 1080 |
| 97 | 1990 | 1515 |
| 98 | 1280 | 1055 |
| 99 | 2080 | 1290 |
| 100 | 1030 | 905 |
| 101 | 2260 | 1460 |
| Vergleichssubstrat* | 490 | 820 |

* Bz-Ile-Glu($\gamma$-OH)-Gly-Arg-pNA · HCl (beschrieben in der DE-OS Nr. 2552570).

Die in Tabelle 4 angegebenen Werte der Faktor-Xa-Aktivität wurden experimentell wie folgt ermittelt:

Zur Bestimmung der Aktivität von bovinem Faktor Xa wurde 1,8 ml Trisimidazolpuffer mit pH 8,4 und Ionenstärke 0,3 bei 37°C mit 0,025 ml einer wässerigen Lösung des Faktor-Xa-Präparates Diagen (Lieferant: Diagnostic Reagents Ltd., Thame, Grossbritannien), welche durch Lösen des Inhalts einer Ampulle in 0,5 ml Wasser erhalten wurde, gut gemischt. Der Mischung wurde 0,2 ml einer $2 \times 10^{-3}$-molaren wässerigen Lösung eines erfindungsgemässen Substrates zugesetzt. Dann wurde die pro Minute freigesetzte Menge des Spaltproduktes $H-R^5$ in Nanomol ermittelt und auf 1 ml Faktor-Xa-Lösung umgerechnet.

Zur Bestimmung der Aktivität von humanem Faktor Xa wurde 0,01 ml Normalzitratplasma mit 0,20 ml einer Lösung von Russel-Viper-Gift in Trisimidazolpuffer mit pH 8,4 und Ionenstärke 0,3, welche 15 mmol $CaCl_2$ pro Milliliter enthielt, gemischt. Die Mischung wurde während 75 s bei 37°C inkubiert, und den im Plasma enthaltenen Faktor X zu aktivieren und vollständig in Faktor Xa überzuführen. Dem Inkubat wurden zuerst 1,40 ml Trisimidazolpuffer (pH 8,4, Ionenstärke 0,3) von 37°C und dann 0,40 ml einer $2 \times 10^{-3}$-molaren wässerigen Lösung eines erfindungsgemässen Substrates zugesetzt. Dann wurde die pro Minute freigesetzte Menge des Spaltproduktes $H-R^5$ in Nanomol ermittelt und auf 1 ml Zitratplasma umgerechnet.

## Patentansprüche

1. Tripeptidderivate der Formel

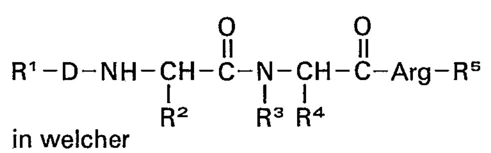

(I)

in welcher

$R^1$ eine gegebenenfalls in $\omega$-Stellung eine Aminogruppe tragende Alkanoylgruppe mit 2 bis 8 Kohlenstoffatomen, eine Phenylalkanoylgruppe, die 2 bis 4 Kohlenstoffatome im Alkanoyl enthält und deren Phenylrest gegebenenfalls in p-Stellung mit einer Aminogruppe substituiert ist, eine gegebenenfalls in 4-Stellung mit einem Aminomethylrest substituierte Cyclohexylcarbonylgruppe, eine gegebenenfalls in o- oder p-Stellung mit Methyl, Amino oder Halogen substituierte Benzoylgruppe, eine Alkoxycarbonylgruppe mit 1 bis 8 Kohlenstoffatomen in der Alkoxygruppe, eine gegebenfalls in p-Stellung mit Methoxy, Methyl oder Chlor substituierte Benzyloxycarbonylgruppe, eine Alkansulfonylgruppe mit 1 bis 4 Kohlenstoffatomen, eine gegebenenfalls in p-Stellung methylierte Phenylsulfonylgruppe oder eine $\alpha$- oder $\beta$-Naphthylsulfonylgruppe darstellt,

$R^2$ einen geradkettigen oder verzweigten Alkylrest mit 1 bis 6 Kohlenstoffatomen, einen Hydroxyalkylrest mit 1 bis 2 Kohlenstoffatomen, einen Alkoxyalkylrest mit 1 bis 4 Kohlenstoffatomen im

Alkoxy, einen Benzyloxyalkylrest mit 1 bis 2 Kohlenstoffatomen im Alkyl, einen ω-Carboxyalkyl- oder ω-Alkoxycarbonylalkylrest mit 1 bis 3 Kohlenstoffatomen im Alkyl, wobei die Alkoxygruppe geradkettig oder verzweigt ist und 1 bis 4 Kohlenstoffatome enthält, oder einen ω-Benzyloxycarbonylalkylrest mit 1 bis 3 Kohlenstoffatomen im Alkyl, oder einen Cyclohexyl-, Cyclohexylmethyl-, 4-Hydroxycyclohexylmethyl-, Phenyl-, Benzyl-, 4-Hydroxybenzyl- oder benzyloder Imidazolyl-(4)-methylrest darstellt,

$R^3$ Wasserstoff oder einen geradkettigen oder verzweigten Alkylrest mit 1 bis 4 Kohlenstoffatomen darstellt,

$R^4$ Wasserstoff oder einen Methyl- oder Äthylrest darstellt, und

$R^5$ eine p-Nitrophenylamino-, 1-Carboxy-2-nitrophenyl-(5)-amino-, 1-Sulfo-2-nitrophenyl-(5)-amino-, β-Naphthylamino-, 4-Methoxy-β-naphthylamino-, 5-Nitro-α-naphthylamino-, Chinonyl-(5)-amino-, 8-Nitrochinoyl-(5)-amino-, 4-Methylcumaryl-(7)-amino- oder 1,3-Di(methoxycarbonyl)phenyl-(5)-aminogruppe (abgeleitet von 5-Aminoisophthalsäuredimethylester) darstellt, ausgenommen Tripeptidderivate, in welchen $R^1$ Benzyloxycarbonyl und $R^2$ Isopropyl oder Cyclohexyl bedeuten, und Salze davon mit Säuren.

2. Tripeptidderivate nach Patentanspruch 1, dadurch gekennzeichnet, dass die stark basische Guanidinogruppe des Arginins durch Protonisierung mit einer Mineralsäure oder einer organischen Säure stabilisiert ist.

3. Tripeptidderivate nach den Patentansprüchen 1 und 2, dadurch gekennzeichnet, dass die Guanidinogruppe des Arginins durch Protonisierung mit Essigsäure stabilisiert ist.

4. Tripeptidderivate nach Patentanspruch 1: Cbo-D-Leu-Gly-Arg-pNA · AcOH, Cbo-D-Leu-Sar-Arg-pNA · AcOH, Cbo-D-Ph'Gly-Gly-Arg-pNA · AcOH, Cbo-D-Nleu-Gly-Arg-pNA · AcOH, Cbo-D-Nleu-Sar-Arg-pNA · AcOH, Cbo-D-Nval-Gly-Arg-pNA · AcOH, Cbo-D-CHA-Gly-Arg-pNA · AcOH, Cbo-D-CHA-Sar-Arg-pNA · AcOH, Cbo-D-CHT-Gly-Arg-pNA · AcOH, Cbo-D-CHT-Sar-pNA·AcOH, $CH_3SO_2$-D-Nleu-Gly-Arg-pNA· AcOH, Isobutoxy-CO-D-Nleu-Gly-Arg-pNA · AcOH, BOC-D-Leu-Gly-Arg-pNA · HBr, 4-MeO-Cbo-D-Leu-Gly-Arg-pNA · HBr, $CH_3O$-CO-D-CHA-Gly-Arg-pNA · AcOH, $C_2H_5O$-CO-D-CHA-Gly-Arg-pNA · AcOH, $CH_3SO_2$-D-CHA-Gly-Arg-pNA · AcOH, 4-Me-Cbo-D-Leu-Gly-Arg-pNA · AcOH, 4-Cl-Cbo-D-Leu-Gly-Arg-pNA · AcOH, BOC-D-(α)-AOA-Gly-Arg-pNA · AcOH.

5. Verfahren zur quantitativen Bestimmung von Faktor Xa in einem Medium, welches Faktor Xa enthält oder in welchem Faktor Xa entsteht oder verbraucht wird, dadurch gekennzeichnet, dass man das genannte Medium mit einem Tripeptidderivat gemäss Patentanspruch 1 zur Reaktion bringt und die durch die katalytische hydrolytische Wirkung des Faktors Xa auf das Tripeptidderivat pro Zeiteinheit freigesetzte Menge des farbigen oder fluoreszierenden Spaltproduktes $H-R^5$ durch photometrische, spektrophotometrische, fluoreszenspektrophotometrische oder elektrochemische Methoden misst.

6. Verfahren nach Patentanspruch 5, dadurch gekennzeichnet, dass man Blutplasma von Menschen oder Warmblütlern mit Gift der Russel-Viper (RVV) oder einem Thromboplastinreagenz behandelt, um den im Plasma enthaltenen inaktiven Faktor X quantitativ in den aktiven Faktor Xa überzuführen, das aktivierte Plasma in einem Puffersystem mit dem genannten Tripeptidderivat zur Reaktion bringt und die durch Einwirkung des Faktors Xa auf das Tripeptidderivat pro Zeiteinheit freigesetzte Menge des Spaltproduktes $H-R^5$ bestimmt.

7. Verfahren zur quantitativen Bestimmung von Heparin über Faktor Xa in heparinisiertem Blutplasma, dadurch gekennzeichnet, dass man in einem Puffersystem eine bekannte Menge Faktor Xa mit dem Blutplasma inkubiert, die Menge des nicht neutralisierten Faktors Xa durch Umsetzung desselben mit einem Tripeptidderivat gemäss Patentanspruch 1 und Bestimmung der pro Zeiteinheit freigesetzten Menge des Spaltproduktes $H-R^5$ misst und aus der Differenz zwischen der eingesetzten Menge Faktor Xa und der Restmenge Faktor Xa die Heparinmenge ermittelt.

8. Verfahren zur quantitativen Bestimmung von Antifaktor Xa in menschlichem Blutplasma über Faktor Xa, dadurch gekennzeichnet, dass man einerseits eine Testprobe herstellt, indem man menschliches Zitratplasma mit einer Pufferlösung verdünnt, der verdünnten Lösung Heparin und dann Faktor Xa beimischt und der Mischung ein Tripeptidderivat gemäss Patentanspruch 1 zugibt, dass man andererseits eine Blindprobe in der gleichen Weise herstellt, indem man jedoch anstelle des verdünnten Plasmas die gleiche Volumenmenge Puffer verwendet, und dass man für beide Proben die durch Bildung des Spaltproduktes $H-R^5$ bewirkte Zunahme der optischen Dichte pro Minute bestimmt und aus der Differenz zwischen der Zunahme der optischen Dichte der Blindprobe pro Minute und der Zunahme der optischen Dichte der Testprobe pro Minute die Menge des durch den Antifaktor-Xa/Heparin-Komplex gehemmten Faktors Xa und daraus die im Plasma vorhandene Menge Antifaktor Xa ermittelt.

9. Verfahren nach einem der Patentansprüche 5, 6, 7 oder 8, dadurch gekennzeichnet, dass man das Spaltprodukt $H-R^5$, wenn $R^5$ eine p-Nitrophenylamino-, 1-Carboxy-2-nitrophenyl-(5)-amino, 1-sulfo-2-nitrophenyl-(5)-amino-, 5-Nitro-α-naphthylamino- oder 8-Nitrochinonyl-(5)-aminogruppe ist, vor der Messung durch Kupplung mit einer Diazoverbindung in eine intensiver gefärbte Verbindung überführt.

**Revendications**

1. Dérivés tripeptidiques de formule

$$R^1-D-NH-\underset{\substack{|\\R^2}}{CH}-\overset{\substack{O\\ \|}}{C}-\underset{\substack{|\\R^3}}{N}-\underset{\substack{|\\R^4}}{CH}-\overset{\substack{O\\ \|}}{C}-Arg-R^5 \qquad (I)$$

dans laquelle

$R^1$ représente un groupe alcanoyle, lequel contient 2 à 8 atomes de carbone et peut porter un groupe amino en position ω, un groupe phénylalcanoyle contenant 2 à 4 atomes de carbone dans l'alcanoyle et dont le radical phényle peut être substitué avec un groupe amino en position para, un groupe cyclohexylcarbonyle, lequel peut être substitué avec un radical aminométhyle en position 4, un groupe benzoyle, lequel peut être substitué avec méthyle, amino ou halogène en position ortho ou para, un groupe alcoxycarbonyle contenant 1 à 8 atomes de carbone dans le radical alcoxy, un groupe benzyloxycarbonyle, lequel peut être substitué en position para avec méthoxy, méthyle ou chlore, un groupe alcanesulfonyle contenant 1 à 4 atomes de carbone, un groupe phénylsulfonyle, lequel peut être méthylé en position para, ou un groupe α- ou β-naphtylsulfonyle,

$R^2$ représente un radical alcoyle linéaire ou ramifié contenant 1 à 6 atomes de carbone, un radical hydroxyalcoyle contenant 1 à 2 atomes de carbone, un radical alcoxyalcoyle contenant 1 à 2 atomes de carbone dans l'alcoyle et 1 à 4 atomes de carbone dans l'alcoxy, un radical benzyloxyalcoyle contenant 1 à 2 atomes de carbone dans l'alcoyle, un radical ω-carboxyalcoyle ou ω-alcoxycarbonylalcoyle contenant 1 à 3 atomes de carbone dans l'alcoyle, le groupe alcoxy pouvant être linéaire ou ramifié et contenant 1 à 4 atomes de carbone, ou un radical ω-benzyloxycarbonylalcoyle contenant 1 à 3 atomes de carbone dans l'alcoyle, ou un radical cyclohexyle, cyclohexylméthyle, 4-hydroxycyclohexylméthyle, phényle, benzyle, 4-hydroxybenzyle ou imidazolyl-(4)-méthyle,

$R^3$ représente l'hydrogène ou un radical alcoyle linéaire ou ramifié contenant 1 à 4 atomes de carbone, $R^4$ représente l'hydrogène ou un radical méthyle ou éthyle, et

$R^5$ représente un groupe p-nitrophénylamino, 1-carboxy-2-nitrophényl-(5)-amino, 1-sulfo-2-nitrophényl-(5)-amino, β-naphtylamino, 4-méthoxy-β-naphthylamino, 5-nitro-α-naphtylamino, quinonyl-(5)-amino, 8-nitroquinonyl-(5)-amino, 4-méthylcoumaryl-(7)-amino ou 1,3-di(méthoxycarbonyl)phényl-(5)-amino (dérivé de l'ester diméthylique de l'acide 5-amino-isophtalique), à l'exception des dérivés tripeptidiques dans lesquels $R^1$ représente benzyloxycarbonyle et $R^2$ représente isopropyle ou cyclohexyle, et leurs sels avec des acides.

2. Dérivés tripeptidiques selon la revendication 1, caractérisés en ce que le groupe guanidino fortement basique de l'arginine est stabilisé par protonisation avec un acide minéral ou organique.

3. Dérivés tripeptidiques selon les revendications 1 et 2, caractérisés en ce que le groupe guanidino de l'arginine est stabilisé par protonisation avec de l'acide acétique.

4. Dérivés tripeptidiques selon la revendication 1: Cbo-D-Leu-Gly-Arg-pNA · AcOH, Cbo-D-Leu-Sar-Arg-pNA · AcOH, Cbo-D-Ph'Gly-Gly-Arg-pNA · AcOH, Cbo-D-Nleu-Gly-Arg-pNA · AcOH, Cbo-D-Nleu-Sar-Arg-pNA · AcOH, Cbo-D-Nval-Gly-Arg-pNA · AcOH, Cbo-D-CHA-Gly-Arg-pNA · AcOH, Cbo-D-CHA-Sar-Arg-pNA · AcOH, Cbo-D-CHT-Gly-Arg-pNA · AcOH, Cbo-D-CHT-Sar-pNA · AcOH, $CH_3SO_2$-D-Nleu-Gly-Arg-pNA · AcOH, Isobutoxy-CO-D-Nleu-Gly-Arg-pNA · AcOH, BOC-D-Leu-Gly-Arg-pNA · HBr, 4-MeO-Cbo-D-Leu-Gly-Arg-pNA · HBr, $CH_3O$-CO-D-CHA-Gly-Arg-pNA · AcOH, $C_2H_5O$-CO-D-CHA-Gly-Arg-pNA · AcOH, $CH_3SO_2$-D-CHA-Gly-Arg-pNA · AcOH, 4-Me-Cbo-D-Leu-Gly-Arg-pNA · AcOH, 4-Cl-Cbo-D-Leu-Gly-Arg-pNA · AcOH, BOC-D-(α)-AOA-Gly-Arg-pNA · AcOH.

5. Procédé pour le dosage quantitatif du facteur Xa dans un milieu comprenant le facteur Xa ou dans lequel le facteur Xa est formé ou consommé, caractérisé en ce qu'on fait réagir ledit milieu avec un dérivé tripeptidique selon la revendication 1 et qu'on mesure, par des méthodes photométriques, spectrophotométriques, spectrophotométriques de fluorescence ou électrochimiques, la quantité du produit de scission coloré ou fluorescent $H-R^5$, libéré par unité de temps par l'action catalytique hydrolytique du facteur Xa sur le dérivé tripeptidique.

6. Procédé selon la revendication 5, caractérisé en ce qu'on traite du plasma sanguin humain ou d'animaux à sang chaud avec du venin de la vipère de Russel (RVV) ou un réactif thromboplastique afin de transformer quantitativement le facteur X inactif présent dans le plasma en facteur Xa actif, qu'on fait réagir le plasma activé dans un système tampon avec ledit dérivé tripeptidique et qu'on détermine la quantité du produit de scission $H-R^5$ libéré par unité de temps par l'action du facteur Xa sur le dérivé tripeptidique.

7. Procédé pour le dosage quantitatif de l'héparine dans du plasma sanguin héparinisé, par l'intermédiaire du facteur Xa, caractérisé en ce qu'on incube une quantité connue de facteur Xa dans un système tampon avec le plasma sanguin, qu'on mesure la quantité du facteur Xa non neutralisée par réaction de ce dernier avec un dérivé tripeptidique selon la revendication 1 et par détermination de la quantité du produit de scission $H-R^5$ libéré par unité de temps, et qu'on détermine la quantité d'héparine à partir de la différence entre la quantité de facteur Xa initialement utilisée et la quantité résiduelle de facteur Xa.

8. Procédé pour le dosage quantitatif de l'anti-facteur Xa dans le plasma sanguin humain par l'intermédiaire du facteur Xa, caractérisé en ce que, d'une part, on prépare un échantillon d'essai en diluant du plasma humain citraté avec une solution tampon, en ajoutant de l'héparine et du facteur Xa à la solution diluée et en additionnant le mélange d'un dérivé tripeptidique selon la revendication 1, et que, d'autre part, on prépare un échantillon témoin de la même manière, mais en utilisant la même quantité volumétrique de tampon au lieu de plasma dilué, et qu'on détermine pour les deux échantillons l'augmentation de la densité optique par minute provoquée par la formation du produit de scission $H-R^5$, et qu'on détermine, à partir de

la différence entre l'augmentation de la densité optique de l'échantillon témoin d'essai par minute et de l'augmentation de la densité optique de l'échantillon d'essai par minute, la quantité du facteur Xa inhibée par le complexe antifacteur-Xa-héparine et par là la quantité d'antifacteur Xa présente dans le plasma.

9. Procédé selon les revendications 5, 6, 7 ou 8, caractérisé en ce que, lorsque $R^5$ représente un groupe p-nitrophénylamino, 1-carboxy-2-nitrophényl-(5)-amino, 1-sulfo-2-nitrophényl-(5)-amino, 5-nitro-α-naphtylamino ou 8-nitroquinonyl-(5)-amino, on transforme le produit de scission $H-R^5$, avant la mesure de sa quantité, en un composé plus intensément coloré par couplage avec un composé diazoïque.

## Claims

1. Tripeptide derivatives having the formula

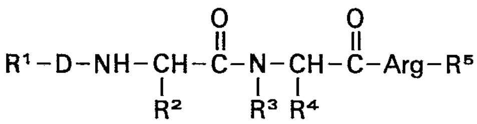

(I)

wherein

$R^1$ represents an alkanoyl group comprising 2 to 8 carbon atoms, which may possibly carry an amino group in the ω-position, a phenylalkanoyl group comprising 2 to 4 carbon atoms in the alkanoyl and whose phenyl radical is possibly substituted with an amino group in the p-position, a cyclohexylcarbonyl group which can possibly be substituted with an aminomethyl radical in the 4-position, a benzoyl group which can possibly be substituted with methyl, amino or halogen in the o- or p-position, an alkoxycarbonyl group comprising 1 to 8 carbon atoms in the alkoxy group, a benzyloxycarbonyl group which can possibly be substituted with methoxy, methyl or chlorine in the p-position, an alkanesulfonyl group comprising 1 to 4 carbon atoms, a phenylsulfonyl group which can possibly be methylated in the p-position, or an α- or β-naphthylsulfonyl group,

$R^2$ represents a straight chain or branched alkyl radical comprising 1 to 6 carbon atoms, a hydroxyalkyl radical comprising 1 to 2 carbon atoms, an alkoxyalkyl radical comprising 1 to 2 carbon atoms in the alkyl and 1 to 4 carbon atoms in the alkoxy, a benzyloxyalkyl radical comprising 1 to 2 carbon atoms in the alkyl, an ω-carboxyalkyl or ω-alkoxycarbonylalkyl radical comprising 1 to 3 carbon atoms in the alkyl, whereby the alkoxy groups is straight-chained or branched and comprises 1 to 4 carbon atoms, or an ω-benzyloxycarbonylalkyl radical comprising 1 to 3 carbon atoms in the alkyl, or a cyclohexyl, cyclohexylmethyl, 4-hydroxycyclohexylmethyl, phenyl, benzyl, 4-hydroxybenzyl or imidazolyl-(4)-methyl radical,

$R^3$ represents hydrogen or a straight chain or branched alkyl radical comprising 1 to 4 carbon atoms,

$R^4$ represents hydrogen or a methyl or ethyl radical, and

$R^5$ represents a p-nitrophenylamino, 1-carboxy-2-nitrophenyl-(5)-amino, 1-sulfo-2-nitrophenyl-(5)-amino, β-naphthylamino, 4-methoxy-β-naphthylamino, 5-nitro-α-naphthylamino, quinonyl-(5)-amino, 8-nitroquinonyl-(5)-amino, 4-methylcoumaryl-(7)-amino or 1,3-di(methoxycarbonyl)phenyl-(5)-amino group (derived from dimethyl 5-amino-isophthalate), with the exception of tripeptide derivatives in which $R^1$ represents benzyloxycarbonyl and $R^2$ represents isopropyl or cyclohexyl, and salts thereof with acids.

2. Tripeptide derivatives according to claim 1, characterized by the fact that the strongly basic guanidino group of arginine is stabilized by protonation with a mineral acid or an organic acid.

3. Tripeptide derivatives according to claims 1 and 2, characterized by the fact that the guanidino group of arginine is stabilized by protonation with acetic acid.

4. Tripeptide derivatives according to claim 1: Cbo-D-Leu-Gly-Arg-pNA · AcOH, Cbo-D-Leu-Sar-Arg-pNA · AcOH, Cbo-D-Ph'Gly-Gly-Arg-pNA · AcOH, Cbo-D-Nleu-Gly-Arg-pNA · AcOH, Cbo-D-Nleu-Sar-Arg-pNA · AcOH, Cbo-D-Nval-Gly-Arg-pNA · AcOH, Cbo-D-CHA-Gly-Arg-pNA · AcOH, Cbo-D-CHA-Sar-Arg-pNA · AcOH, Cbo-D-CHT-Gly-Arg-pNA · AcOH, Cbo-D-CHT-Sar-pNA·AcOH, $CH_3SO_2$-D-Nleu-Gly-Arg-pNA· AcOH, Isobutoxy-CO-D-Nleu-Gly-Arg-pNA · AcOH, BOC-D-Leu-Gly-Arg-pNA · HBr, 4-MeO-Cbo-D-Leu-Gly-Arg-pNA · HBr, $CH_3O$-CO-D-CHA-Gly-Arg-pNA · AcOH, $C_2H_5O$-CO-D-CHA-Gly-Arg-pNA · AcOH, $CH_3SO_2$-D-CHA-Gly-Arg-pNA · AcOH, 4-Me-Cbo-D-Leu-Gly-Arg-pNA · AcOH, 4-Cl-Cbo-D-Leu-Gly-Arg-pNA · AcOH, BOC-D-(α)-AOA-Gly-Arg-pNA · AcOH.

5. Method for quantitatively assaying factor Xa in a medium which contains factor Xa or in which factor Xa is formed or consumed, characterized by the fact that the said medium is reacted with a tripeptide derivative according to claim 1, and that the quantity of the coloured or fluorescent split product $H-R^5$ released per time unit by the catalytic hydrolytic action of factor Xa on the tripeptide derivative is measured by photometric, spectrophotometric, fluorescence spectrophotometric or electrochemical methods.

6. Method according to claim 5, characterized by treating blood plasma of humans or warm-blooded animals with Russel viper venom (RVV) or a thromboplastin reagent in order to quantitatively convert the inactive factor X present in the plasma into the active factor Xa, reacting the activated plasma in a buffer system with the said tripeptide derivative, and measuring the quantity of split product $H-R^5$ released per time unit by the action of factor Xa on the tripeptide derivative.

7. Method for quantitatively assaying heparin in heparinized blood plasma *via* factor Xa, characterized by incubating a known quantity of factor Xa with blood plasma in a buffer system, measuring

the quantity of non-neutralized factor Xa by reacting the latter with a tripeptide derivative according to claim 1 and assaying the quantity of split product $H-R^5$ released per time unit, and calculating the heparin level from the difference between the quantity of factor Xa initially used and the residual amount of factor Xa.

8. Method for quantitatively assaying antifactor Xa in human blood plasma *via* factor Xa, characterized by preparing, on the one hand, a test sample by diluting human citrate plasma with a buffer solution, adding heparin and then factor Xa to the diluted solution and admixing a tripeptide derivative according to claim 1 therewith, preparing, on the other hand, a blank test sample in the same manner, but using the same volumetric amount of buffer instead of dilute plasma, and determining for both samples the increase in the optical density per minute caused by the formation of split product $H-R^5$, and determining the quantity of factor Xa inhibited by the antifactor-Xa-heparin complex and therefrom the quantity of antifactor Xa initially present in the plasma from the difference of the increase in the optical density of the blank test sample per minute and the increase in the optical density of the test sample per minute.

9. Method according to claims 5, 6, 7, or 8, characterized by the fact that, if $R^5$ is a p-nitrophenylamino, 1-carboxy-2-nitrophenyl-(5)-amino, 1-sulfo-2-nitrophenyl-(5)-amino, 5-nitro-α-naphthylamino or 8-nitroquinonyl-(5)-amino group, the split product $H-R^5$ is converted into a more intensely coloured compound by coupling with a diazo compound before the measurement of its quantity.